(19) Europäisches Patentamt
European Patent Office
Office européen des brevets

(11) EP 0 779 276 A1

# (12) EUROPÄISCHE PATENTANMELDUNG

(43) Veröffentlichungstag:
**18.06.1997 Patentblatt 1997/25**

(21) Anmeldenummer: **96119320.8**

(22) Anmeldetag: **03.12.1996**

(51) Int. Cl.$^6$: **C07D 209/42**, C07D 235/16, C07D 235/18, C07D 471/04, A61K 31/415

(84) Benannte Vertragsstaaten:
**AT BE CH DE DK ES FI FR GB GR IE IT LI LU MC NL PT SE**

(30) Priorität: **15.12.1995 DE 19546919**

(71) Anmelder: **BAYER AG**
**51368 Leverkusen (DE)**

(72) Erfinder:
- **Connell, Richard, Dr.**
  **Trumbull, CT 06611 (US)**
- **Goldmann, Siegfried, Dr.**
  **42327 Wuppertal (DE)**
- **Müller, Ulrich, Dr.**
  **42111 Wuppertal (DE)**
- **Lohmer, Stefan, Dr.**
  **20133 Milano (IT)**
- **Bischoff, Hilmar, Dr.**
  **42113 Wuppertal (DE)**
- **Denzer, Dirk, Dr.**
  **42115 Wuppertal (DE)**
- **Grützmann, Rudi, Dr.**
  **42657 Solingen (DE)**
- **Wohlfeil, Stefan, Dr.**
  **40721 Hilden (DE)**

## (54) Indolyl-substituierte Phenylessigsäurederivate

(57) Die Indolyl-substituierten Phenylessigsäurederivate werden hergestellt, indem man die entsprechenden Phenylessigsäuren mit den erforderlichen Aminen umsetzt. Die Indolyl-substituierten Phenylessigsäurederivate eignen sich als Wirkstoffe in Arzneimitteln, insbesondere in antiarteriosklerotischen Arzneimitteln.

EP 0 779 276 A1

## Beschreibung

Die vorliegende Erfindung betrifft Indolyl-substituierte Phenylessigsäurederivate, Verfahren zu ihrer Herstellung und ihre Verwendung als Arzneimittel, insbesondere als antiatherosklerotische Arzneimittel.

Heterocyclisch substituierte Phenylessigsäurederivate und substituierte Imidazo[4,5-b]pyridine und Benzimidazole sind aus den Publikationen DOS 42 00 954 und DOS 43 02 956 bekannt. Außerdem sind aus der Publikation US 5 314 880 Benzimidazol- Derivate mit einer PAF antagonistischen Wirkung beschrieben.

Die erfindungsgemäßen Verbindungen werden partiell vom Bedeutungsumfang dieser Publikationen umfaßt.

Es ist bekannt, daß erhöhte Blutspiegel von Triglyceriden (Hypertriglyceridämie) und Cholesterin (Hypercholesterinämie) mit der Genese von atherosklerotischen Gefäßwand-Veränderungen und koronaren Herzkrankheiten assoziiert sind.

Ein deutlich erhöhtes Risiko für die Entwicklung koronarer Herzerkrankungen liegt darüberhinaus vor, wenn diese beiden Risikofaktoren kombiniert auftreten, was wiederum mit einer Überproduktion an Apoliprotein B-100 einhergeht. Es ist daher nach wie vor ein starkes Bedürfnis, wirksame Arzneimittel zur Bekämpfung der Atherosklerose sowie koronarer Herzkrankheiten zur Verfügung zu stellen.

Die vorliegende Erfindung betrifft Indolyl-substituierte Phenylessigsäurederivate der allgemeinen Formel (I)

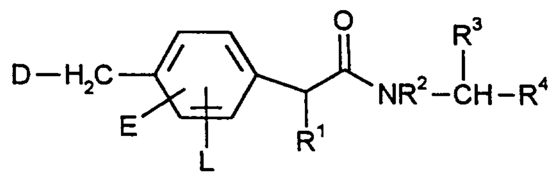

(I)

in welcher

D für einen Rest der Formel

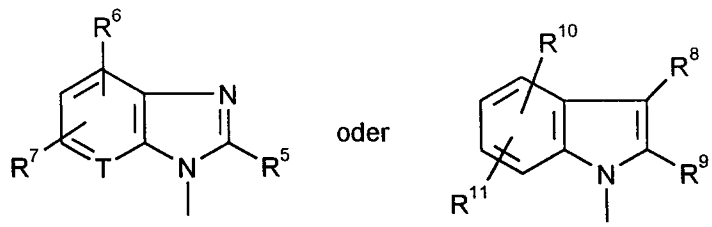

steht,

worin

T ein Stickstoffatom oder die -CH-Gruppe bedeutet,

$R^6$, $R^7$, $R^{10}$ und $R^{11}$ gleich oder verschieden sind und Wasserstoff, Trifluormethyl, Halogen oder geradkettiges oder verzweigtes Alkyl oder Alkoxy mit jeweils bis zu 6 Kohlenstoffatomen bedeuten,

$R^5$, $R^8$ und $R^9$ gleich oder verschieden sind und Wasserstoff, Cycloalkyl mit 3 bis 6 Kohlenstoffatomen, Phenyl, geradkettiges oder verzweigtes Alkoxycarbonyl mit bis zu 6 Kohlenstoffatomen oder geradkettiges oder verzweigtes Alkyl mit bis zu 6 Kohlenstoffatomen bedeuten, das gegebenenfalls durch Halogen substituiert ist,

oder im Fall, daß T für ein Stickstoffatom steht, $R^5$ auch Benzyl bedeuten kann,

E und L gleich oder verschieden sind und für Wasserstoff, Halogen, Trifluormethyl, Hydroxy, Carboxyl oder für geradkettiges oder verzweigtes Alkyl, Alkoxy oder Alkoxycarbonyl mit jeweils bis zu 6 Kohlenstoffatomen stehen,

$R^1$ für Cycloalkyl mit 3 bis 10 Kohlenstoffatomen oder
für geradkettiges oder verzweigtes Alkyl mit 1 bis 10 Kohlenstoffatomen steht, oder
für Phenyl steht, das gegebenenfalls bis zu 2-fach gleich oder verschieden durch Halogen, Cyano, Hydroxy, geradkettiges oder verzweigtes Alkyl oder Alkoxy mit jeweils bis zu 4 Kohlenstoffatomen substituiert ist,

$R^2$ für Wasserstoff oder geradkettiges oder verzweigtes Alkyl mit bis zu 3 Kohlenstoffatomen steht,

$R^3$ für Wasserstoff oder geradkettiges oder verzweigtes Alkyl mit bis zu 5 Kohlenstoffatomen steht, oder
für Cycloalkyl mit 3 bis 7 Kohlenstoffatomen steht, oder
für Phenyl oder für einen 5- bis 7-gliedrigen aromatischen Heterocyclus mit bis zu 3 Heteroatomen aus der Reihe S, N und/oder O steht, die gegebenenfalls bis zu 3-fach gleich oder verschieden durch Halogen, Nitro, Phenyl, Hydroxy oder durch geradkettiges oder verzweigtes Alkyl oder Alkoxy mit bis zu 6 Kohlenstoffatomen substituiert sind,

$R^4$ für Wasserstoff oder für eine Gruppe der Formel $-CH_2-OH$ oder $CH_2O-CO-R^{12}$ steht,

worin

$R^{12}$ Wasserstoff, geradkettiges oder verzweigtes Alkyl mit bis zu 8 Kohlenstoffatomen oder Phenyl bedeutet, das gegebenenfalls bis zu 3-fach gleich oder verschieden durch Halogen, Hydroxy, Cyano oder geradkettiges oder verzweigtes Alkyl oder Alkoxy mit jeweils bis zu 4 Kohlenstoffatomen substituiert ist,

und deren Salze.

Die erfindungsgemäßen heterocyclisch substituierten Phenylessigsäurederivate und substituierten Imidazo[4,5-b]pyridine und Benzimidazole können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di-bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Diese Mischungen der Enantiomeren und Diastereomeren lassen sich in bekannter Weise in die stereoisomer einheitlichen Bestandteile trennen.

Heterocyclus, gegebenenfalls benzokondensiert, steht im Rahmen der Erfindung im allgemeinen für einen gesättigten oder ungesättigten 5- bis 7-gliedrigen, vorzugsweise 5- bis 6-gliedrigen Heterocyclus der bis zu 3 Heteroatome aus der Reihe S, N und/oder O enthalten kann und der im Fall eines Stickstoffatoms auch über dieses gebunden sein kann. Beispielsweise seien genannt: Indolyl, Chinolyl, Benzo[b]thienyl, Benzo[b]furyl, Pyridyl, Thienyl, Furyl, Pyrrolyl, Thiazolyl, Oxazolyl, Imidazolyl, Morpholinyl oder Piperidyl. Bevorzugt sind Chinolyl, Furyl, Pyridyl und Thienyl.

Bevorzugt sind Verbindungen der allgemeinen Formel (I),
in welcher

D für einen Rest der Formel

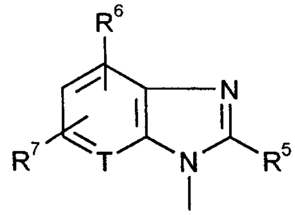

oder

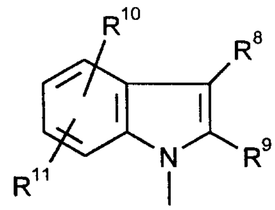

steht,

worin

T ein Stickstoffatom oder die -CH-Gruppe bedeutet,

$R^6$, $R^7$, $R^{10}$ und $R^{11}$ gleich oder verschieden sind und Wasserstoff, Trifluormethyl, Fluor, Chlor, Brom oder geradkettiges oder verzweigtes Alkyl oder Alkoxy mit jeweils bis zu 5 Kohlenstoffatomen bedeuten,

$R^5$, $R^8$ und $R^9$ gleich oder verschieden sind und Wasserstoff, Cyclopropyl, Cyclopentyl, Cyclohexyl, Phenyl, geradkettiges oder verzweigtes Alkoxycarbonyl mit bis zu 5 Kohlenstoffatomen oder geradkettiges oder verzweigtes Alkyl mit bis zu 5 Kohlenstoffatomen bedeuten, das gegebenenfalls durch Fluor, Chlor oder Brom substituiert ist,

oder im Fall, daß T für ein Stickstoffatom steht, $R^5$ auch Benzyl bedeuten kann,

E und L gleich oder verschieden sind und für Wasserstoff, Fluor, Chlor, Brom, Trifluormethyl, Hydroxy oder geradkettiges oder verzweigtes Alkyl oder Alkoxy mit jeweils bis zu 4 Kohlenstoffatomen stehen,

$R^1$ für Cyclobutyl, Cyclopentyl, Cyclohexyl, Cycloheptyl, Cylcooctyl oder für geradkettiges oder verzweigtes Alkyl mit bis zu 7 Kohlenstoffatomen steht, oder für Phenyl steht, das gegebenenfalls durch Fluor, Chlor, Brom, Cyano, Hydroxy oder geradkettiges oder verzweigtes Alkyl oder Alkoxy mit jeweils bis zu 3 Kohlenstoffatomen substituiert ist,

$R^2$ Wasserstoff oder Methyl bedeutet,

$R^3$ für Wasserstoff oder geradkettiges oder verzweigtes Alkyl mit bis zu 4 Kohlenstoffatomen, Cyclopropyl, Cyclopentyl, Cyclohexyl oder für Phenyl, Pyridyl, Thienyl oder Furyl steht, die gegebenenfalls bis zu 2-fach gleich oder verschieden durch Fluor, Chlor, Brom, Phenyl, Nitro, Hydroxy oder durch geradkettiges oder verzweigtes Alkyl oder Alkoxy mit bis zu 4 Kohlenstoffatomen substituiert sind,

$R^4$ für Wasserstoff oder für eine Gruppe der Formel $-CH_2-OH$ oder $-CH_2O-CO-R^{12}$ steht,

worin

$R^{12}$ Wasserstoff, geradkettiges oder verzweigtes Alkyl mit bis zu 6 Kohlenstoffatomen oder Phenyl bedeutet, das gegebenenfalls bis zu 2-fach gleich oder verschieden durch Fluor, Chlor, Brom, Cyano, Hydroxy oder geradkettiges oder verzweigtes Alkyl oder Alkoxy mit jeweils bis zu 3 Kohlenstoffatomen substituiert ist,

und deren Salze.

Besonders bevorzugt sind Verbindungen der allgemeinen Formel (I), in welcher

D für einen Rest der Formel

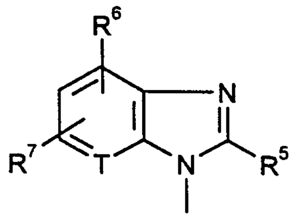

oder

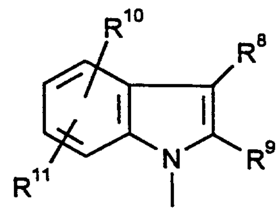

steht,

worin

T ein Stickstoffatom oder die -CH-Gruppe bedeutet,

$R^6$, $R^7$, $R^{10}$ und $R^{11}$ gleich oder verschieden sind und Wasserstoff, Trifluormethyl, Chlor oder geradkettiges oder verzweigtes Alkyl oder Alkoxy mit jeweils bis zu 4 Kohlenstoffatomen bedeuten,

$R^5$, $R^8$ und $R^9$ gleich oder verschieden sind und Wasserstoff, Cyclopropyl, Cyclopentyl, Cyclohexyl, Phenyl, geradkettiges oder verzweigtes Alkoxycarbonyl mit bis zu 4 Kohlenstoffatomen oder geradkettiges oder verzweigtes Alkyl mit bis zu 4 Kohlenstoffatomen bedeuten, das gegebenenfalls durch Chlor substituiert ist,

oder im Fall, daß T für ein Stickstoffatom steht, $R^5$ auch Benzyl bedeuten kann,

E und L gleich oder verschieden sind und für Wasserstoff, Fluor, Chlor, Brom oder Trifluormethyl stehen,

$R^1$ für Cyclopentyl, Cyclohexyl, Cycloheptyl, Cylcooctyl oder für geradkettiges oder verzweigtes Alkyl mit bis zu 6 Kohlenstoffatomen steht, oder für Phenyl steht, das gegebenenfalls durch Fluor, Chlor, Cyano, Hydroxy, Methyl oder Methoxy substituiert ist,

$R^2$ für Wasserstoff oder Methyl steht,

$R^3$ für Wasserstoff oder geradkettiges oder verzweigtes Alkyl mit bis zu 3 Kohlenstoffatomen, Cyclopropyl, Cyclopentyl, Cyclohexyl oder für Pyridyl, Thienyl oder Phenyl steht, die gegebenenfalls bis zu 2-fach gleich oder verschieden durch Fluor, Chlor, Brom, Nitro, Phenyl, Hydroxy oder durch geradkettiges oder verzweigtes Alkyl oder Alkoxy mit bis zu 3 Kohlenstoffatomen substituiert sind,

$R^4$ für Wasserstoff, für eine Gruppe der Formel $-CH_2-OH$ oder $-CH_2O-CO-R^{12}$ steht,

worin

$R^{12}$ Wasserstoff geradkettiges oder verzweigtes Alkyl mit bis zu 6 Kohlenstoffatomen oder Phenyl bedeutet, das gegebenenfalls bis zu 2-fach gleich oder verschieden durch Fluor, Chlor, Brom, Cyano, Hydroxy, Methyl oder Methoxy substituiert ist,

und deren Salze.

Außerdem wurde ein Verfahren zur Herstellung der erfindungsgemäßen Verbindungen der allgemeinen Formel (I) gefunden, dadurch gekennzeichnet, daß man

Verbindungen der allgemeinen Formel (II)

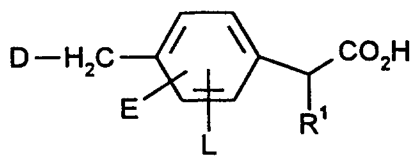

(II)

in welcher

D, E, L und $R^1$ die oben angegebene Bedeutung haben,

mit Verbindungen der allgemeinen Formel (III)

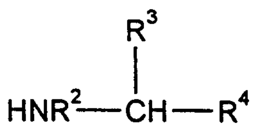

(III)

in welcher

$R^2$, $R^3$ und $R^4$ die oben angegebene Bedeutung haben,

in inerten Lösemitteln, gegebenenfalls in Anwesenheit einer Base und/oder Hilfsstoffes umsetzt.

Das erfindungsgemäße Verfahren kann durch folgendes Formelschema beispielhaft erläutert werden:

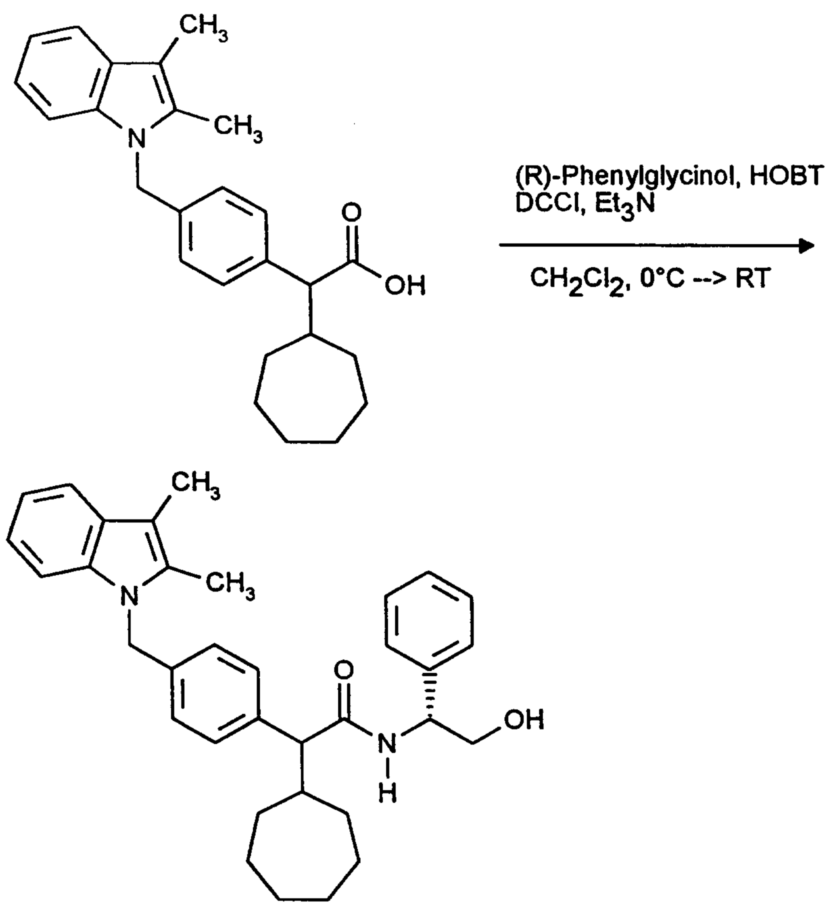


Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dichlormethan, Tetrahydrofuran und Dimethylformamid.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat, Kaliumcarbonat oder Cäsiumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl($C_1$-$C_6$)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle wie Natrium oder deren Hydride wie Natriumhydrid einzusetzen. Bevorzugt sind Natriumhydrid, Kaliumcarbonat, Triethylamin, Trimethylamin, Pyridin, Kalium-tert.butylat, DBU oder DABCO.

Im allgemeinen setzt man die Base in einer Menge von 0,05 Mol bis 10 Mol, bevorzugt von 1 Mol bis 2 Mol bezogen auf 1 Mol der Verbindung der Formel (II) ein.

Die Amidierung kann gegebenenfalls über die aktivierte Stufe der Säurehalogenide oder gemischter Anhydride, die aus den entsprechenden Säuren durch Umsetzung mit Thionylchlorid, Phosphortrichlorid, Phosphorpentachlorid, Phosphortribromid oder Oxalylchlorid oder Methansulfonsäurechlorid hergestellt werden können, verlaufen.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexafluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Das erfindungsgemäße Verfahren wird im allgemeinen in einem Temperaturbereich von -50°C bis +100°C, bevorzugt von -30°C bis +60°C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Verbindungen der allgemeinen Formel (II) sind teilweise bekannt oder neu und können dann beispielsweise durch Umsetzung von Verbindungen der allgemeinen Formel (IV)

D-H (IV)

in welcher

D die oben angegebene Bedeutung hat,

mit Verbindungen der allgemeinen Formel (V)

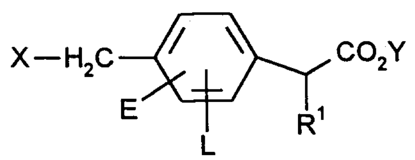

(V)

in welcher

E, L und $R^1$ die oben angegebene Bedeutung haben,

X für Halogen steht,

Y für geradkettiges oder verzweigtes Alkyl mit bis zu 4 Kohlenstoffatomen steht,

in einem der oben aufgeführten Lösemittel und Basen,
und abschließende Überführung der Ester nach üblichen Methoden in die entsprechenden Säuren hergestellt werden.

Als Lösemittel wird bevorzugt Dimethylformamid eingesetzt.

Als Basen wird bevorzugt Natriumhydrid eingesetzt.

Im allgemeinen setzt man die Base in einer Menge von 0,05 Mol bis 10 Mol, bevorzugt von 1 Mol bis 2 Mol bezogen auf 1 Mol der Verbindung der Formel (IV) ein.

Das erfindungsgemäße Verfahren wird im allgemeinen in einem Temperaturbereich von -50°C bis +100°C, bevorzugt von -30°C bis +60°C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Verbindungen der allgemeinen Formel (III), (IV) und (V) sind an sich bekannt oder nach üblichen Methoden herstellbar.

Die erfindungsgemäßen Verbindungen der allgemeinen Formel (I) haben ein nicht vorhersehbares pharmakologisches Wirkspektrum.

Sie können als Wirkstoffe in Arzneimitteln zur Reduzierung von Veränderungen an Gefäßwänden Verwendung finden und zur Behandlung von Koronaren Herzerkrankungen, Herzinsuffizienz, Störungen der Hirnleistung, ischämischen Gehirnerkrankungen, Apoplex, Durchblutungsstörungen, Mikrozirkulationsstörungen und Thrombosen.

Weiterhin spielt bei der Okklusion von Gefäßen die Proliferation glatter Muskelzellen eine ausschlaggebende Rolle. Die erfindungsgemäßen Verbindungen sind geeignet, diese Proliferation zu inhibieren und damit atherosklerotische Prozesse zu verhindern.

Die erfindungsgemäßen Verbindungen zeichnen sich durch eine Senkung der ApoB-100-assoziierten Lipoproteinen (VLDL und seiner Abbauprodukte, wie z.B. LDL), des ApoB-100, der Triglyceride und des Cholesterins aus. Damit besitzen sie wertvolle, im Vergleich zum Stand der Technik überlegene pharmakologische Eigenschaften.

Überraschenderweise besteht die Wirkung der erfindungsgemäßen Verbindungen zunächst in einer Verminderung oder vollständigen Inhibierung der Bildung und/oder der Freisetzung von ApoB-100-assoziierten Lipoproteinen aus Leberzellen, was eine Senkung des VLDL-Plasmaspiegels zur Folge hat. Diese VLDL-Senkung muß mit einer Senkung

der Plasmaspiegel von ApoB-100, LDL, Triglyceriden und von Cholesterin einhergehen; es werden also gleichzeitig mehrere der obengenannten Risikofaktoren gesenkt, die an Gefäßwandveränderungen beteiligt sind.

Die erfindungsgemäßen Verbindungen können daher zur Präventation und Behandlung von Atherosklerose, der Fettsucht, Pankreatitis und der Obstipation eingesetzt werden.

**1. Hemmung der Freisetzung ApoB-100-assoziierter Lipoproteine**

Der Test zum Nachweis der Hemmung der Freisetzung ApoB-100-assoziierter Liproproteine aus Leberzellen erfolgte in vitro mit kultivierten Leberzellen, bevorzugt mit Zellen der humanen Linie HepG2. Diese Zellen werden unter Standardbedingungen in Medium für die Kultur eukariontischer Zellen gezüchtet, bevorzugt in RPMI 1640 mit 10% fötalem Kälberserum. HepG2-Zellen synthetisieren und sezernieren in den Kulturüberstand ApoB-100-assoziierte Lipoproteinpartikel, die im Prinzip ähnlich aufgebaut sind wie die VLDL- bzw. LDL-Partikel, die im Plasma zu finden sind.

Diese Partikel können mit einem Immunoassay für humanes LDL nachgewiesen werden. Dieser Immunoassay erfolgt mit Antikörpern, die im Kaninchen gegen humanes LDL unter Standardbedingungen induziert worden waren. Die anti-LDL-Antikörper (Kan-anti-LDL-Ak) wurden an einem Immunosorbens mit humanem LDL affinitätschromatographisch gereinigt. Diese gereinigten Kan-anti-LDL-Ak werden an die Oberfläche von Plastik adsorbiert. Zweckmäßigerweise erfolgt diese Adsorbtion an die Plastikoberfläche von Mikrotitierplatten mit 96 Vertiefungen, bevorzugt an MaxiSorp-Platten. Wenn im Überstand von Hep-G2-Zellen ApoB-100-assoziierte Partikel vorhanden sind, dann können diese an die insolubilisierten Kan-anti-LDL-Ak binden, und es entsteht ein Immunkomplex, der an die Plastikoberfläche gebunden ist. Nicht gebundene Proteine werden durch Waschen entfernt. Der sich an der Plastikoberfläche befindliche Immunkomplex wird mit monoklonalen Antikörpern nachgewiesen, die nach Standardbedingungen gegen humanes LDL induziert und gereinigt worden waren. Diese Antikörper wurden mit dem Enzym Peroxidase konjugiert. Peroxidase setzt das farblose Substrat TMB in Gegenwart von $H_2O_2$ in ein gefärbtes Produkt um. Nach Ansäuerung des Reaktionsgemisches mit $H_2SO_4$ wird die spezifische Lichtadsorption bei 450 nm bestimmt, die ein Maß für die Menge von ApoB-100-assoziierten Partikeln ist, die von den HepG2-Zellen in den Kulturüberstand sezerniert worden waren.

Überraschenderweise hemmen die erfindungsgemäßen Verbindungen die Freisetzung der ApoB-100-assoziierten Partikel. Der $IC_{50}$-Wert gibt an, bei welcher Substanzkonzentration die Lichtadsorption im Vergleich zur Kontrolle (Lösemittelkontrolle ohne Substanz) um 50% inhibiert ist.

| Bsp.-Nr. | $IC_{50}$ [$10^{-9}$ mol/l] |
|---|---|
| 2 | 29.5 |
| 3 | 62.5 |
| 4 | 40.5 |
| 5 | 83.2 |
| 6 | 37.4 |
| 7 | 228.9 |
| 8 | 50.5 |
| 11 | 489.2 |
| 12 | 10.8 |
| 13 | 24.6 |
| 14 | 473.5 |
| 15 | 47.3 |
| 16 | 35.9 |
| 17 | 38.1 |
| 20 | 8.1 |
| 21 | 4.5 |
| 22 | 23.5 |
| 23 | 36.1 |
| 26 | 26.8 |
| 27 | 6.5 |
| 28 | 62.6 |
| 29 | 40.5 |
| 30 | 128.5 |
| 31 | 428.3 |

| Bsp.-Nr. | $IC_{50}$ [$10^{-9}$ mol/l] |
|---|---|
| 32 | 48.5 |
| 33 | 41.1 |
| 34 | 12.3 |
| 35 | 308.0 |
| 36 | 72.5 |
| 37 | 31.1 |
| 38 | 269.7 |
| 39 | 29.4 |
| 40 | 93.6 |
| 41 | 40.4 |
| 42 | 9.3 |
| 43 | 5.0 |
| 44 | 130.6 |
| 45 | 302.4 |
| 46 | 58.9 |
| 47 | 78.6 |
| 48 | 491.2 |
| 49 | 78.6 |
| 50 | 420.6 |
| 51 | 78.6 |
| 52 | 58.9 |
| 53 | 491.2 |
| 54 | 56.7 |
| 55 | 18.9 |
| 56 | 170.1 |
| 57 | 10.8 |
| 58 | 1.5 |
| 59 | 26.9 |
| 60 | 9.5 |
| 62 | 151.9 |
| 63 | 1010.1 |
| 64 | 150.9 |

| Bsp.-Nr. | $IC_{50}$ [$10^{-9}$ mol/l] |
|---|---|
| 66 | 8.1 |
| 67 | 30.2 |
| 69 | 9.6 |

**2. Bestimmung der VLDL-Sekretion in vivo am Hamster**

Der Effekt der Testsubstanzen auf die VLDL-Sekretion in vivo wird am Hamster untersucht. Hierzu werden Goldhamster nach Prämedikation mit Atropin (83 mg/kg s.c.) mit Ketavet (83 mg/kg s.c.) und Nembutal (50 mg/kg i.p.) narkotisiert. Wenn die Tiere reflexfrei geworden sind, wird die V. jugularis freipräpariert und kanüliert. Anschließend werden 0,25 ml/kg einer 20%igen Lösung von Triton WR-1339 in physiologischer Kochsalzlösung appliziert. Dieses Detergens hemmt die Lipoproteinlipase und führt so zu einem Anstieg des Triglyceridspiegels aufgrund eines ausbleibenden Katabolismus von sezernierten VLDL-Partikeln. Dieser Triglyceridanstieg kann als Maß für die VLDL-Sekretionsrate herangezogen werden. Den Tieren wird vor sowie ein und zwei Stunden nach Applikation des Detergens durch Punktion des retroorbitalen Venenplexus Blut entnommen. Das Blut wird zwei Stunden bei Raumtemperatur, anschließend über Nacht bei 4°C inkubiert, um die Gerinnung vollständig abzuschließen. Danach wird 5 Minuten bei 10.000 g zentrifugiert. Im so erhaltenen Serum wird die Triglyceridkonzentration mit Hilfe eines modifizierten kommerziell erhältlichen Enzymtests bestimmt (Merckotest® Triglyceride Nr. 14354). 100 µl Serum werden mit 100 µl Testreagenz in 96-Lochplatten versetzt und 10 Minuten bei Raumtemperatur inkubiert. Anschließend wird die optische Dichte bei einer Wellenlänge von 492 nm in einem automatischen Platten-Lesegerät bestimmt (SLT-Spectra). Serumproben mit einer zu hohen Triglyceridkonzentration werden mit physiologischer Kochsalzlösung verdünnt. Die in den Proben enthaltene Triglyceridkonzentration wird mit Hilfe einer parallel gemessenen Standardkurve bestimmt. Testsubstanzen werden in diesem Modell entweder unmittelbar vor Applikation des Detergens intravenös verabreicht oder vor Einleitung der Narkose oral oder subkutan.

**3. Hemmung der intestinalen Triglyceridabsoprtion in vivo (Ratten)**

Die Substanzen, die auf ihre triglyceridabsorptionshemmende Wirkung in vivo untersucht werden sollen, werden männlichen Wistar-Ratten mit einem Körpergewicht zwischen 170 und 230 g oral verabreicht. Zu diesem Zweck werden die Tiere 18 Stunden vor der Substanzapplikation in Gruppen zu 6 Tieren eingeteilt und anschließend wird ihnen das Futter entzogen. Trinkwasser steht den Tieren ad libitum zur Verfügung. Die Tiere der Kontrollgruppen erhalten eine wäßrige Traganth-Suspension bzw. eine Traganth-Suspension die Olivenöl enthält. Die Traganth-Olivenöl-Suspension wird mit dem Ultra-Turrax hergestellt. Die zu untersuchenden Substanzen werden in einer entsprechenden Traganth-Olivenöl-Suspension ebenfalls mit dem Ultra-Turrax, direkt vor der Substanzapplikation suspendiert.

Jeder Ratte wird vor der Schlundsondenapplikation zur Bestimmung des basalen Serumtriglyceridgehaltes Blut durch Punktion des retroorbitalen Venenplexus' entnommen. Anschließend werden die Traganth-Suspension, die Traganth-Olivenöl-Suspensionen ohne Substanz (Kontrolltiere), bzw. die Substanzen, suspendiert in einer entsprechenden Traganth-Olivenöl-Suspension, den nüchternen Tieren mit einer Schlundsonde verabreicht. Die weiteren Blutentnahmen zur Bestimmung des postprandialen Serumtriglyceridanstiegs erfolgen in der Regel 1, 2 und 3 Stunden nach der Schlundsondenapplikation.

Die Blutproben werden zentrifugiert und nach Gewinnung des Serums die Triglyceride photometrisch mit einem EPOS-Analyzer 5060 (Eppendorf Gerätebau, Netheler & Hinz GmbH, Hamburg) bestimmt. Die Bestimmung der Trigylceride erfolgt vollenzymatisch mit einem handelsüblichen UV-Test.

Der postprandiale Serumtriglyceridanstieg wird durch Subtraktion des Triglyceridvorwertes jeden Tieres von seinen korrespondierenden postprandialen Triglyceridkonzentrationen (1, 2 und 3 Stunden nach Applikation) ermittelt.

Die Differenzen (in mmol/l) zu jedem Zeitpunkt (1, 2 und 3 Stunden) werden in den Gruppen gemittelt, und die Mittelwerte des Serumtriglyceridanstiegs (ΔTG) der substanzbehandelten Tiere mit den Tieren verglichen, die nur die Traganth-Öl-Suspension erhielten.

Ebenso wird der Serumtriglyceridverlauf der Kontrolltiere, die nur Traganth erhielten, berechnet. Der Substanzeffekt zu jedem Zeitpunkt (1, 2 oder 3 Stunden) wird wie folgt ermittelt und in Δ% von der ölbelasteten Kontrolle angegeben.

$$\Delta\%\ \text{Triglyceridanstieg} = \frac{\Delta TG_{\text{Substanz}} - \Delta TG_{\text{Traganthkontrolle}}}{\Delta TG_{\text{Ölbelastung}} - \Delta TG_{\text{Traganthkontrolle}}} \times 100$$

Effekt von 10 mg Prüfsubstanz / kg KG p.o. auf den Triglyceridanstieg (Δ%) 2 h nach einer Triglyceridbelastung im Serum nüchterner Ratten. Der Serumtriglyceridanstieg fettbelasteter Kontrolltiere bezogen auf den Serumtriglyceridspiegel von Traganth-Kontrolltieren entspricht 100%. n = 6 Tiere pro Gruppe.

Die statistische Auswertung erfolgt mit Student's t-Test nach vorheriger Überprüfung der Varianzen auf Homogenität.

Substanzen, die zu einem Zeitpunkt den postprandialen Serumtriglyceridanstieg, verglichen mit dem der unbehandelten Kontrollgruppe, statistisch signifikant ($p < 0{,}05$) um mindestens 30 % vermindern, werden als pharmakologisch wirksam angesehen.

| Bsp.-Nr. | TG Absorption $ED_{50}$ oder % Inhibition mg/kg/p.o. |
|---|---|
| 2 | < 3 mg/kg |
| 4 | < 10 mg/kg |
| 12 | << 3 mg/kg |
| 13 | < 3 mg/kg |
| 16 | > 2 mg/kg |
| 20 | >> 10 mg/kg |
| 21 | >> 6 mg/kg |
| 23 | 2 mg/kg |
| 27 | > 2 mg/kg |
| 29 | 3 mg/kg |
| 34 | 2 mg/kg |
| 37 | > 2 mg/kg |
| 41 | 10 mg/kg |
| 42 | > 2 mg/kg |
| 43 | 10 mg/kg |
| 46 | 30 mg/kg |
| 51 | 40 mg/kg |
| 54 | 50 mg/kg |
| 57 | > 3 mg/kg |
| 60 | 10 mg/kg |
| 67 | > 3 mg7kg |

**4. Hemmung der VLDL-Sekretion in vivo (Ratte)**

Die Wirkung der Testsubstanzen auf die VLDL-Sekretion wird ebenfalls an der Ratte untersucht. Dazu wird Ratten 500 mg/kg Körpergewicht (2,5 mg/kg) Triton WR-1339, gelöst in physiologischer Kochsalzlösung, intravenös in die Schwanzvene appliziert. Triton WR-1339 inhibiert die Lipoproteinlipase und führt somit durch Hemmung des VLDL-Katabolismus zu einem Anstieg des Triglycerid- und Cholsterinspiegels. Diese Anstiege können als Maß für die VLDL-Sekretionsrate herangezogen werden.

Den Tieren wird vor sowie eine und zwei Stunden nach Applikation des Detergens durch Punktion des retroorbitalen Venenplexus Blut entnommen. Das Blut wird zur Gerinnung 1 h bei Raumtemperatur inkubiert und das Serum durch Zentrifugation mit 10 000 g für 20 s gewonnen. Anschließend werden die Triglyceride mittels eines handelsüblichen gekoppelten Enzymtests (Sigma Diagnostics®, Nr. 339) bei einer Wellenlänge von 540 nm photometrisch bestimmt. Die Messung erfolgt mit Hilfe eines ebenfalls gekoppelten Enzymtests (Boehringer Mannheim®, Nr. 1442350) bei einer Wellenlänge von 546 nm. Proben mit Triglycerid- bzw. Cholesterinkonzentrationen, die den Meßbereich der Methoden überschreiten, werden mit physiologischer Kochsalzlösung verdünnt. Die Ermittlung der jeweiligen Serumkonzentrationen erfolgt anhand parallel gemessener Standardreihen. Testsubstanzen werden unmittelbar nach der Tritoninjektion oral, intravenös oder subcutan appliziert.

Die Erfindung betrifft außerdem die Kombination von heterocyclisch substituierten Phenylessigsäurederivaten und substituierten Imidazo[4.5-b]pyridine und Benzimidazole der allgemeinen Formel (I) mit einem Glucosidase- und/oder Amylasehemmer zur Behandlung von familiärer Hyperlipidämien, der Fettsucht (Adipositas) und des Diabetes mellitus. Glucosidase- und/oder Amylasehemmer im Rahmen der Erfindung sind beispielsweise Acarbose, Adiposine, Voglibose (AO-128), Miglitol, Emiglitate, MDL-25637, Camiglibase (MDL-73945), Temdamistate, AI-3688, Trestatin, Pradimicin-Q und Salbostatin. Bevorzugt ist die Kombination von Acarbose, Miglitol, Emiglitate oder Vaglibose mit einer der oben aufgeführten erfindungsgemäßen Verbindungen der allgemeinen Formel (I).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

**Verwendete Abkürzungen:**

| | |
|---|---|
| bs = | breites Singulett |
| CI = | Chemische Ionisation |
| cHept = | cyclo Heptyl |
| cHex = | cyclo Hexyl |
| cPent = | cyclo Pentyl |
| DCCI = | N'(3-Dimethylaminopropyl)-N'-ethylcarbodiimid |
| d = | Dublett |
| dia = | Diastereomer |
| dd = | Dublett von Dubletts |
| DMF = | N,N-Dimethylformamid |
| DMSO = | Dimethylsulfoxid |
| EI = | Elektronenstoßionisation |
| FAB = | Fast Atom Bombardment |
| HOBT = | 1-Hydroxy-1H-benzotriazol |
| Hz = | Hertz |
| iBu = | iso Butyl |
| iPr = | iso Propyl |
| m = | Multiplett |
| Me = | Methyl |

nPr = normal Propyl
Ph = Phenyl
RT = Raumtemperatur
s = Singulett
t = Triplett
TFA = Trifluoressigsäure
THF = Tetrahydrofuran
TMS = Tetramethylsilan

**Benutzte Solvenzgemische**

| | |
|---|---|
| Petrolether : Aceton | = 1:1 (A) |
| Petrolether : Essigester | = 20:1 (B) |
| Petrolether : Essigester | = 10:1 (C) |
| Petrolether : Essigester | = 5:1 (D) |
| Petrolether : Essigester | = 3:1 (E) |
| Petrolether : Essigester | = 4:1 (F) |
| Petrolether : Essigester | = 2:1 (G) |
| Petrolether : Essigester | = 1:1 (H) |
| Petrolether : Essigester | = 1:2 (I) |
| Dichlormethan : Methanol | = 50:1 (J) |
| Dichlormethan : Methanol | = 20:1 (K) |
| Dichlormethan : Methanol | = 10:1 (L) |
| Dichlormethan : Essigester | = 1:1 (M) |
| Dichlormethan : Ethanol | = 50:1 (N) |
| Dichlormethan (100%) | = (O) |
| Essigester : Methanol | = 10:1 (P) |
| Toluol (100%) | = (Q) |
| Toluol : Essigester | = 1:1 (R) |
| Toluol : Essigester | = 8:1 (S) |
| Toluol : Essigester | = 9:1 (T) |
| Cyclohexanol : Essigester | = 1:1 (U) |
| Cyclohexanol : Essigester | = 7:3 (V) |

<u>**Ergänzungen:**</u>

Wenn FAB nicht benutzt worden ist, haben folgende Kennzeichnungen in allen nachfolgenen Tabellen Gültigkeit:

* = EI
# = CI ($NH_3$)

**Beispiel I**

2-(R&S)-Phenyl-2-(4-methyl)phenylessigsäuremethylester

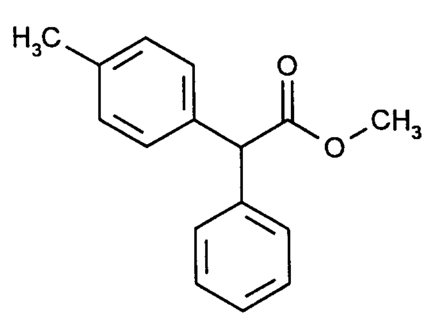

21.0 g (100 mmol, Apin) 2-Phenyl-1-(4-methyl)phenyl-1-oxoethan und 38.8 g (120 mmol) Iodbenzoldiacetat wurden in 300 ml Orthoameisensaeuretrimethylester gelöst. Zu dieser Lösung wurden 19.6 g konz. Schwefelsäure gegeben, und die Lösung wurde für 6 Std auf 60°C erwärmt. Die Lösung wurde auf Raumtemperatur abgekühlt, mit Wasser (100 ml) verdünnt, und mit Diethylether extrahiert. Die vereinigten organischen Phasen wurden über Natriumsulfat getrocknet und einrotiert. Der Rückstand wurde säulenchromatographisch gereinigt:
Ausbeute 13.1 g (55%);
$R_f$ = 0.33 (Petrolether : Essigester = 20 : 1);
Masse (berechnet) für $C_{16}H_{16}O_2$ = 240.30; Massenspektrum (FAB, rel. Intensität) 241 (25%), 181 (100%);
$^1H$ NMR (200 MHz, $CDCl_3$) δ 7.3-7.10 (m, 9 H), 4.99 (s, 1 H), 3.73 (s, 3 H), 2.31 (s, 3 H).

**Beispiel II**

2-Cyclopentyl-2-(4-methylphenyl)-essigsäure-tert.butylester

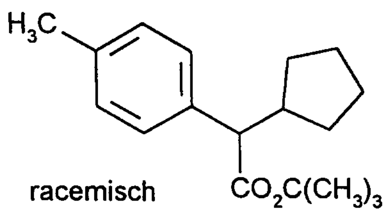

racemisch

33.5 g (0.3 mol) Kalium-tert.butylat werden in 100 ml wasserfreiem DMF bei 0°C vorgelegt, und 51.6 g (0.25 mol) 4-Methylphenyl-essigsäure-tert.butylester in 250 ml wasserfreiem DMF zugetropft. Es wird 30 min. bei 0°C gerührt und 32.2 ml (0.3 mol) Cyclopentylbromid in 150 ml wasserfreiem DMF bei 5-15°C zugetropft und 20 h bei 25°C gerührt. Nach Einengen wird der Rückstand zwischen Wasser und Diethylether verteilt, die Etherphase über Natriumsulfat getrocknet und eingeengt. Das Produkt kristallisiert aus.
Ausbeute: 67 g (97.5% d.Th.);
Festpunkt: 51-53°C.

Die Verbindungen der Tabelle I werden analog der Vorschrift des Beispiels II hergestellt:

**Tabelle I:**

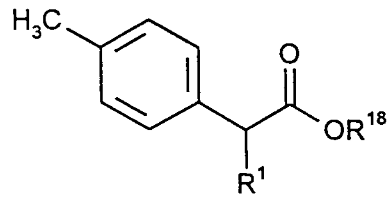

| Bsp.-Nr. | $R^1$ | $R^{18}$ | $R_f$ * |
|---|---|---|---|
| III | (R&S) iPr | Me | 0.86 (T) |
| IV | (R&S) iBu | tBu | 0.84 (S) |
| V | (R&S) cPent | Me | 0.59 (C) |
| VI | (R&S) cHex | Me | 0.38 (B) |
| VII | (R&S) cHex | tBu | 0.71 (Q) |
| VIII | (R&S) cHept | Me | 0.57 (Q) |
| IX | (R&S) cHept | tBu | 0.32 (Q) |

**Beispiel X**

2-(4-Brommethyl-phenyl)-2-cyclopentyl-essigsäure-tert.butylester

racemisch

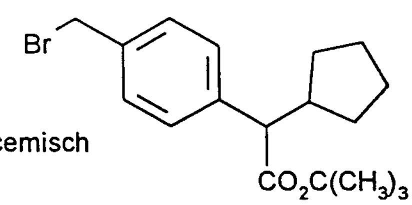

27.4 g (0.1 mol) der Verbindung aus Beispiel II werden in 200 ml Tetrachlormethan gelöst und zum Sieden erhitzt. Nach Zugabe von 0.82 g Azobisisobutyronitril werden 18.7 g (0.105 mol) N-Bromsuccinimid portionsweise zugegeben und anschließend 1 h refluxiert, auf 0°C abgekühlt und vom Succinimid abfiltriert. Nach Einengen des Filtrats fällt das Produkt aus. Es wird mit Petrolether (40/60) gewaschen und getrocknet.
Ausbeute: 20 g (57% d.Th.);
Fp.: 73-76°C.

Die Verbindungen der Tabelle II werden analog der Vorschrift des Beispiels X hergestellt:

## Tabelle II

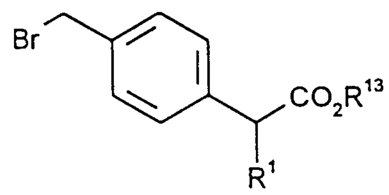

| Bsp.-Nr. | $R^1$ | $R^{13}$ | $R_f$ * |
|---|---|---|---|
| XI | (R&S) iPr | Me | 0.78 (O) |
| XII | (R&S) iBu | tBu | 0.86 (O) |
| XIII | (R&S) cPent | Me | 0.63 (C) |
| XIV | (R&S) cHex | Me | 0.74 (O) |
| XV | (R&S) cHex | tBu | 0.58 (C) |
| XVI | (R&S) cHept | tBu | 0.84 (O) |
| XVII | (R&S) Ph | Me | 0.74 (O) |

**Beispiel XVIII**

4-Methyl-2-propyl-1H-benzimidazol

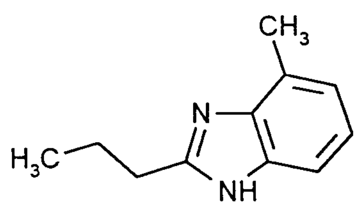

5.00 g (41 mmol) 2,3-Diamino-toluol und 3.7 ml (41 mmol) Buttersäure werden 3 h bei 120°C in 41 ml Polyphosphorsäure gerührt, in 410 ml Wasser gegeben und mit festem Natriumhydrid auf pH = 9 gestellt. Darauf setzt man bis zu Beendigung der Kohlendioxidenwicklung Natriumcarbonat zu, extrahiert fünfmal mit je 100 ml Essigsäureethylester, trocknet die vereinigten organsichen Phasen mit wasserfreiem Natriumsulfat und dampft die Lösung - zuletzt im Hochvakuum - ein; Rohauswaage: 5.89 g.

Das Produkt wird chromatographisch an Kieselgel 60 (Merck / 40 - 63 µm / Dichlormethan : Methanol = 100:1) gereinigt; Ausbeute: 2.45 (34%).

$R_f$ = 0.16 (Dichlormethan : Methanol = 20 : 1)

In Analogie zur Vorschrift des Beispiels XVIII werden die in der Tabelle III aufgeführten Verbindungen hergestellt:

## Tabelle III:

| Bsp.-Nr. | D | Ausbeute (% d.Th.) | $R_f$ (Solvens) | Fp. (°C) | MS (EI) (rel. Intensität) |
|---|---|---|---|---|---|
| XIX | 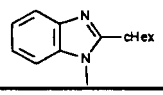 | 97 | 0.85 (P) | >240 | 200 (30%), 145 (100%) |
| XX | 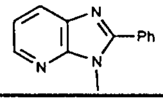 | 81 | 0.66 (P) | >220 | 199 (100%) |
| XXI | 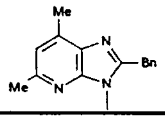 | 27 | 0.39 (K) | 184 | 238 (100%) |
| XXII | 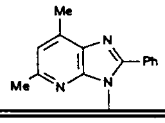 | 23 | 0.39 (K) | 240 | #224 (100%) |

**Beispiel XXIII**

2-Cycloheptyl-2-[4-(2,3-dimethylindol-1-yl-methyl)phenyl] essigsäure-tert.butylester

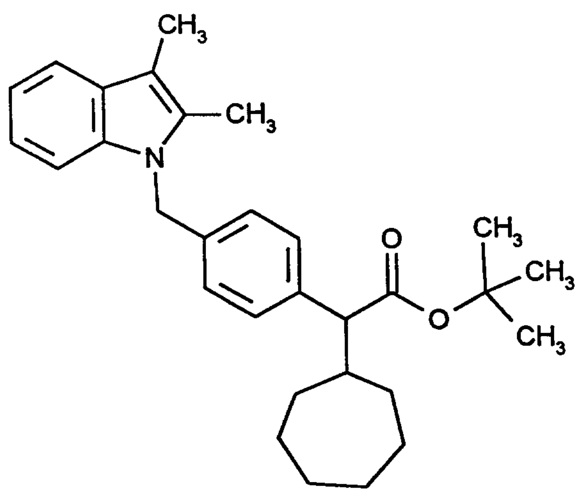

Es wird 1.0 g (6.2 mmol) 2,3-Dimethylindol in 10 ml DMF gelöst. Die Lösung wird unter Argon auf 0°C abgekühlt. Dazu gibt man portionsweise 0.32 g (8.0 mmol) Natriumhydrid (60%ig in Paraffin). Es wird eine halbe Stunde bei 0°C nachgerührt. Eine Lösung von 3.5 g (6.2 mmol) XVI in 40 ml DMF wird langsam zugetropft. Man rührt über Nacht bei Raumtemperatur nach. DMF wird über Hochvakuum abdestilliert und der Rückstand in Wasser und Essigester verteilt. Die organische Phase wird über Natriumsulfat getrocknet und einrotiert. Der Rückstand wird säulenchromatographisch gereinigt (Kieselgel Merck 60 (0.040-0.063)):
Ausbeute: 1.3 g (42.3 %);
Fp = 121-123°C;
$R_f$ = 0.42 (Petrolether : Essigester = 20 : 1);

Masse (berechnet) für $C_{30}H_{39}NO_2$ = 445.65: Massenspektrum (DCI ($NH_3$), rel. Intensität) 445 (100%), 389 (30%); $^1$H NMR (250 MHz, $CDCl_3$) δ 7.52 (m, 1 H), 7.22-7.18 (m, 3 H), 7.15-7.05 (m, 2 H), 6.89 (d, J = 8.22 Hz, 2 H), 5.25 (s, 2 H), 3.12 (d, J = 10.83 Hz, 1 H), 2.28 (s, 3 H), 2.27 (s, 3 H), 2.12 (m, 1 H), 1.84-1.20 (m, 11 H), 1.36 (s, 9 H), 0.90 (m, 1 H).

In Analogie zur Vorschrift des Beispiels XXIII werden die in der Tabelle IV aufgeführten Verbindungen hergestellt:

Tabelle IV

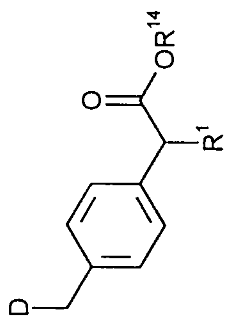

| Bsp.-Nr. | D | $R^{14}$ | $R^1$ | Ausbeute (% d.Th.) | Rf (Solvens) | Fp. (°C) | MS (*) (rel. Intensität) |
|---|---|---|---|---|---|---|---|
| XXIV | 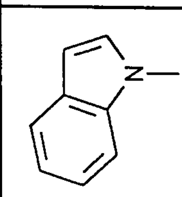 | tBu | (R&S) cHept | 48 | 0.43 (B) | 123-124 | # 418 (100%) |
| XXV | 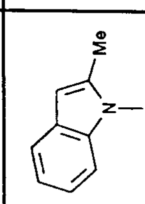 | tBu | (R&S) cHept | 49 | 0.43 (B) | 124-26 | * 431 (100%), 57 (70%) |
| XXVI | 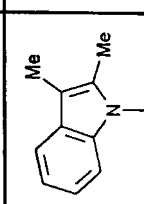 | tBu | (R&S) cPent | 24 | 0.25 (B) | 115 | 417 (100%), 360 (20%) |
| XXVI | 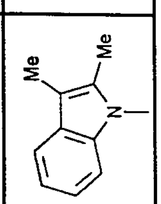 | Me | (R&S) cHex | 55 | 0.35 (K) | 98-101 | 389 (100%) |

| Bsp.-Nr. | D | $R^{14}$ | $R^1$ | Ausbeute (% d.Th.) | Rf (Solvens) | Fp. (°C) | MS (*) (rel. Intensität) |
|---|---|---|---|---|---|---|---|
| XXVIII | 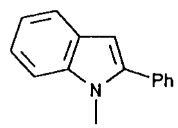 | tBu | (R&S) cHept | 39 | 0.39 (B) | | * 493 (20%), 57 (100%) |
| XXIX | 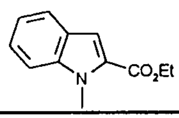 | tBu | (R&S) cPent | 48 | 0.46 (C) | 86 | 461 (85%), 406 (100%) |
| XXX | 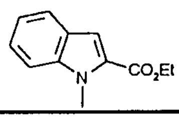 | tBu | (R&S) cHept | 43 | 0.32 (B) | | * 489 (60%), 57 (100%) |
| XXXI | 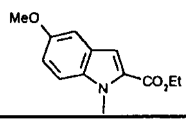 | tBu | (R&S) cPent | 42 | 0.58 (O) | 106 | 491 (100%), 436 (40%), 57 (60%) |
| XXXII | 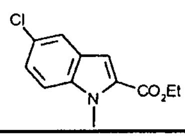 | tBu | (R&S) cPent | 64 | 0.25 (L) | 100-02 | 495 (70%), 440 (100%), 57 (100%) |
| XXXIII | 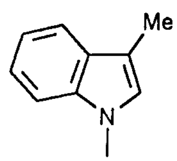 | tBu | (R&S) cHept | 76 | 0.41 (B) | 100-01 | # 431 (20%), 375 (100%) |
| XXXIV | 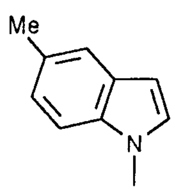 | tBu | (R&S) cPent | 65 | 0.42 (B) | | 403 (20%), 346 (40%) |

| Bsp.-Nr. | D | $R^{14}$ | $R^1$ | Ausbeute (% d.Th.) | Rf (Solvens) | Fp. (°C) | MS (*) (rel. Intensität) |
|---|---|---|---|---|---|---|---|
| XXXV | 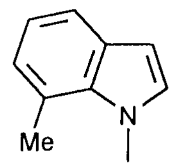 | tBu | (R&S) cPent | 67 | 0.33 (B) | | # 404 (100%) |
| XXXVI | 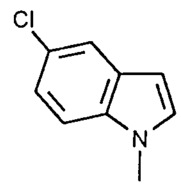 | tBu | (R&S) cHept | 54 | 0.35 (B) | | * 451 (30%), 57 (100%) |
| XXXVII | 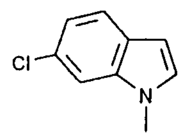 | tBu | (R&S) cPent | 75 | 0.34 (B) | | 423 (100%) |
| XXXVIII | 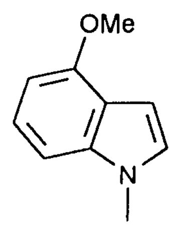 | tBu | (R&S) cPent | 79 | 0.25 (B) | | # 420 (100%) |
| XXXIX | 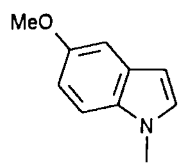 | tBu | (R&S) cHept | 53 | 0.24 (B) | | * 447 (100%), 57 (60%) |

| Bsp.-Nr. | D | $R^{14}$ | $R^1$ | Ausbeute (% d.Th.) | Rf (Solvens) | Fp. (°C) | MS (*) (rel. Intensität) |
|---|---|---|---|---|---|---|---|
| XL | 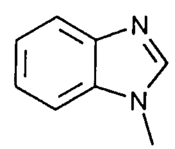 | tBu | (R&S) cHept | 67 | 0.14 (J) | | * 418 (10%), 57 (100%) |
| XLI | 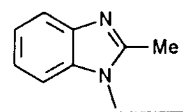 | tBu | (R&S) cHept | 96 | 0.42 (K) | 109 | * 432 (100%) |
| XLII | 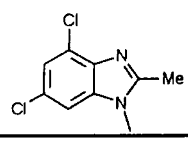 | tBu | (R&S) cPent | 53 | 0.20 (E) | | 475 (80%), 473 (100%) |
| XLIII | 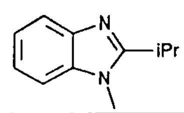 | tBu | (R&S) cPent | 70 | 0.48 (J) | 111 | |
| XLIV | 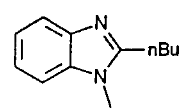 | tBu | (R&S) cPent | 94 | 0.81 (K) | | |
| XLV | 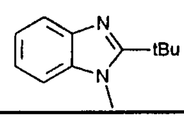 | tBu | (R&S) cPent | 55 | 0.64 (J) | 134 | 447 (100%) |
| XLVI | 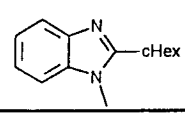 | tBu | (R&S) cPent | 66 | 0.47 (K) | 56 | 473 (100%), 57 (40%) |

| Bsp.-Nr. | D | $R^{14}$ | $R^1$ | Ausbeute (% d.Th.) | Rf (Solvens) | Fp. (°C) | MS (*) (rel. Intensität) |
|---|---|---|---|---|---|---|---|
| XLVII | 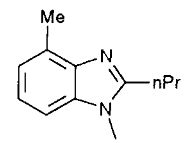 | tBu | (R&S) cPent | 80 | 0.51 (G) | | |
| XLVIII | 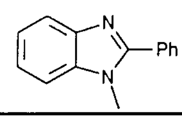 | tBu | (R&S) cPent | 61 | 0.27 (H) | | |
| XLIX | 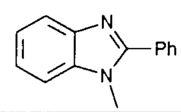 | tBu | (R&S) cHept | 20 | 0.28 (V) | 0:1 | |
| L | 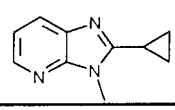 | tBu | (R&S) cPent | 27 | 0.64 (K) | | 432 (40%), 57 (100%) |
| LI | 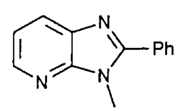 | tBu | (R&S) cPent | 97 | 0.47 (L) | 0:1 | |
| LII | 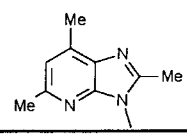 | tBu | (R&S) cPent | 32 | 0.29 (J) | 92 | 434 (100%), 162 (65%) |
| LIII | 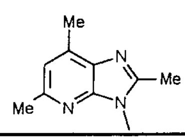 | Me | (R&S) cHex | 29 | 0.59 (K) | 0:1 | 406 (100%) |
| LIV | 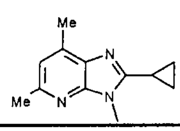 | tBu | (R&S) cPent | 43 | 0.14 (J) | 118 | 460 (100%), 188 (20%) |

| Bsp.-Nr. | D | $R^{14}$ | $R^1$ | Ausbeute (% d.Th.) | Rf (Solvens) | Fp. (°C) | MS (*) (rel. Intensität) |
|---|---|---|---|---|---|---|---|
| LV | 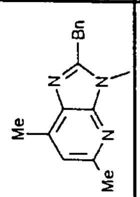 | tBu | (R&S) cPent | 60 | 0.36 (J) | 110 | 510 (100%), 57 (25%) |
| LVI | 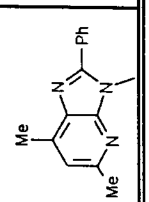 | tBu | (R&S) cPent | 56 | 0.55 (J) 0.83 (K) | | #496 (100%) |

**Beispiel LVII und LVIII**

Man löst 2.30 g (5.0 mmol) der Verbindung aus Beispiel LIV in 30 ml Dioxan und versetzt die Lösung mit 2.3 ml

konzentrierter Salzsäure. Man läßt über Nacht zum Rückfluß kochen, kühlt ab und versetzt mit 30 ml kaltem Wasser. Der entstandene Niederschlag wird abgesaugt und getrocknet:
Ausbeute: 1.68 g.
Da laut NMR und Masse zwei Verbindungen entstanden sind, wurden diese bei HPLC getrennt.

Cyclopentyl-[4-(2-cyclopropyl-5,7-dimethyl-imidazo[4,5-b]pyridin-3-yl)-phenyl]-essigsäure

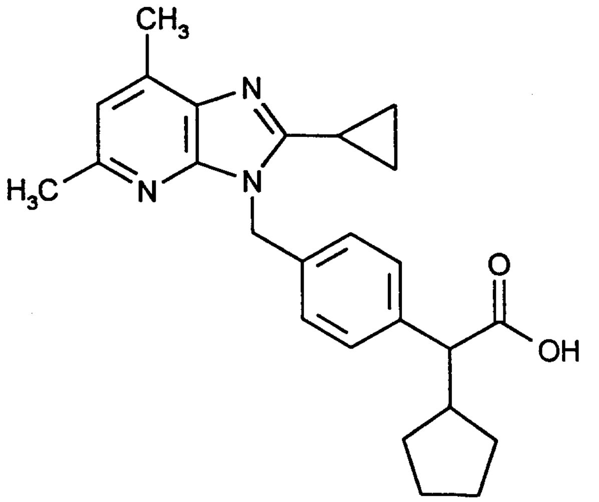

(LVII)

Fp = 180°C;
Rf = 0.24 ($CH_2Cl_2$ : Methanol = 100 : 5);
Masse (berechnet) für $C_{25}H_{29}N_3O_2$ = 403.53: Massenspektrum (FAB, rel. Intensität) 404 (100%).

{4-[2-(3-chloropropyl)-5,7-dimethyl-imidazo[4,5-b]pyridin-3-yl]-phenyl}-cyclopentylessigsäure

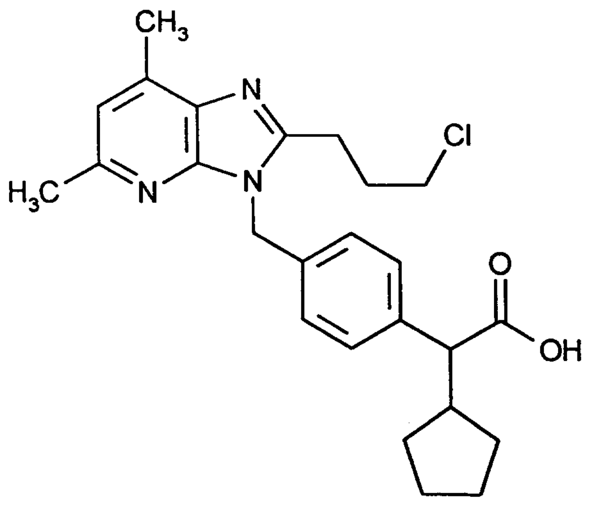

(LVIII)

Fp = 214°C;
Rf = 0.24 ($CH_2Cl_2$ : Methanol = 100 : 5);
Masse (berechnet) für $C_{25}H_{30}ClN_3O_2$ = 439.99: Massenspektrum (FAB, rel. Intensität) 440 (75%).

**Beispiel LIX**

2-Cycloheptyl-2-[4-(2,3-dimethylindol-1-yl-methyl)phenyl] essigsäure

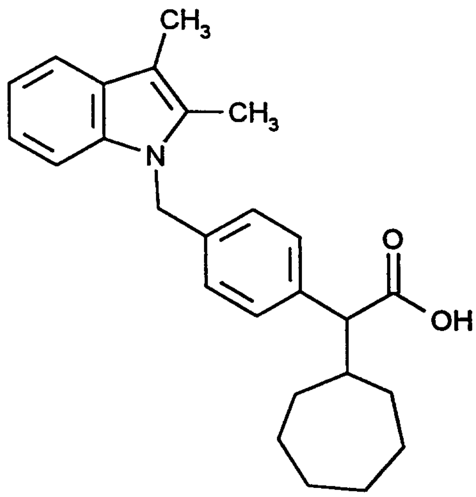

In 15 ml Dioxan wird 1.3 g (2.92 mmol) der Verbindung aus Beispiel XXIII gelöst. Dazu gibt man 1.6 ml konzentrierte Salzsäure und kocht 6 Stunden zum Rückfluß. Man gibt Wasser und $CH_2Cl_2$ dazu und extrahiert. Die organische Phase wird über Natiumsulfat getrocknet und einrotiert. Der Rückstand wird säulenchromatographisch gereinigt (Kieselgel Merck 60 (0.040-0.063)):
Ausbeute: 1.0 g (88.0 %);
$R_f$ = 0.25 (Petrolether : Essigester = 3 : 1);
Masse (berechnet) für $C_{26}H_{31}NO_2$ = 389.54; Massenspektrum (EI, rel. Intensität) 389 (100%), 149 (45%);
$^1H$ NMR (200 MHz, $CDCl_3$) $\delta$ 7.51 (m, 1 H), 7.22-7.05 (m, 5 H), 6.89 (d, J = 8.11 Hz, 2 H), 5.25 (s, 2 H), 3.20 (d, J = 10.88 Hz, 1 H), 2.27 (s, 6 H), 2.18 (m, 1 H), 1.80 (m, 1 H), 1.70-1.17 (m, 10 H), 0.95 (m, 1 H).

Tabelle V

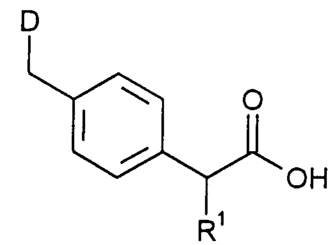

| Bsp.-Nr. | D | $R^1$ | Ausbeute (% d.Th.) | $R_f$ (Solvens) | Fp. (°C) | MS (*) (rel. Intensität) |
|---|---|---|---|---|---|---|
| LX | 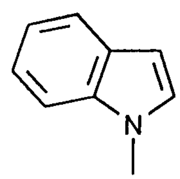 | (R&S) cHept | 72 | 0.52 (R) | | # 362 (100%) |
| LXI | 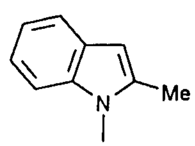 | (R&S) cHept | 77 | 0.53 (R) | | # 376 (100%) |

| Bsp.-Nr. | D | $R^1$ | Ausbeute (% d.Th.) | $R_f$ (Solvens) | Fp. (°C) | MS (*) (rel. Intensität) |
|---|---|---|---|---|---|---|
| LXII | 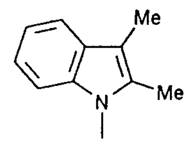 | (R&S) cPent | 80 | 0.26 (E) | 173 | 361 (100%) |
| LXIII | 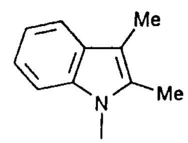 | (R&S) cHex | 87 | 0.51 (K) | 209 | 375 (100%) |
| LXIV | 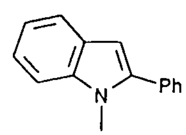 | (R&S) cHept | 49 | 0.28 (K) | | * 437 (100%), 55 (100%) |
| LXV | 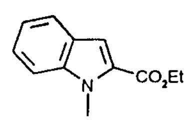 | (R&S) cPent | 72 | 0.47 (K) | 186 | 406 (80%), 405 (100%) |
| LXVI | 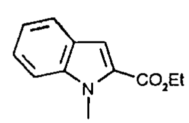 | (R&S) cHept | 68 | 0.29 (K) | | * 433 (100%) |

| Bsp.-Nr. | D | $R^1$ | Ausbeute (% d.Th.) | $R_f$ (Solvens) | Fp. (°C) | MS (*) (rel. Intensität) |
|---|---|---|---|---|---|---|
| LXVII | 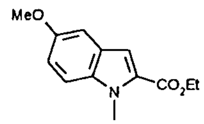 | (R&S) cPent | 79 | 0.48 (K) | 142 | 435 (100%) |
| LXVIII | 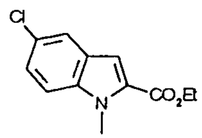 | (R&S) cPent | 94 | 0.48 (K) | 67 (Schaum) | 439 (100%), 217 (80%) |
| LXIX | 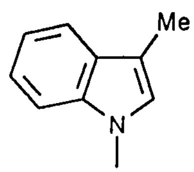 | (R&S) cHept | 78 | 0.27 (E) | | * 395 (100%) |
| LXX | 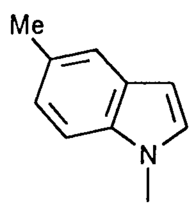 | (R&S) cPent | 64 | 0.44 (K) | Öl | * 347 (80%), 88 (100%) |

| Bsp.-Nr. | D | $R^1$ | Ausbeute (% d.Th.) | $R_f$ (Solvens) | Fp. (°C) | MS (*) (rel. Intensität) |
|---|---|---|---|---|---|---|
| LXXI | 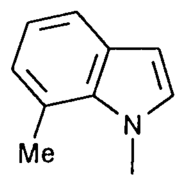 | (R&S) cPent | 68 | 0.62 (K) | | * 347 (60%), 149 (100%) |
| LXXII | 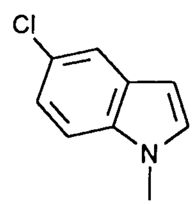 | (R&S) cHept | 70 | 0.33 (K) | | * 395 (80%), 149 (100%) |
| LXXIII | 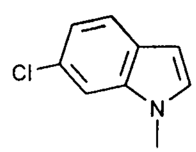 | (R&S) cPent | 88 | 0.51 (K) | Öl | * 367 (100%) |

| Bsp.-Nr. | D | $R^1$ | Ausbeute (% d.Th.) | $R_f$ (Solvens) | Fp. (°C) | MS (*) (rel. Intensität) |
|---|---|---|---|---|---|---|
| LXXIV | 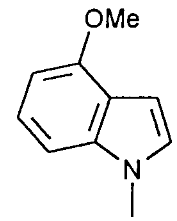 | (R&S) cPent | 92 | 0.44 (K) | | * 363 (100%) |
| LXXV | 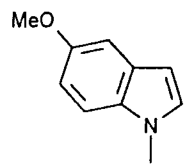 | (R&S) cHept | 63 | 0.37 (K) | | * 391 (100%) |
| LXXVI | 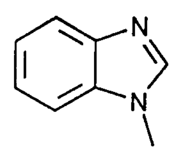 | (R&S) cHept | 68 | 0.18 (K) 0.50 (L) | 123 (Schaum) | # 363 (100%), 362 (90%) |
| LXXVII | 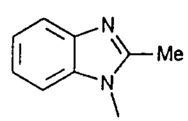 | (R&S) cHept | 100 | 0.16 (K) | 108 | * 376 (100%) |

| Bsp.-Nr. | D | $R^1$ | Ausbeute (% d.Th.) | $R_f$ (Solvens) | Fp. (°C) | MS (*) (rel. Intensität) |
|---|---|---|---|---|---|---|
| LXXVIII | 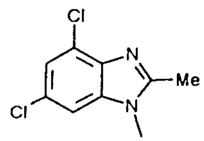 | (R&S) cPent | 100 | 0.07 (K) | >220 | 419 (60%), 417 (100%) |
| LXXIX | 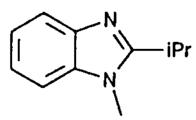 | (R&S) cPent | 80 | 0.32 (K) | 234 | 377 (100%) |
| LXXX | 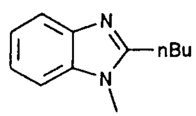 | (R&S) cPent | 92 | 0.44 (K) | | |
| LXXXI | 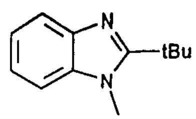 | (R&S) cPent | 88 | 0.27 (K) | 126 (Schaum) | 391 (100%) |
| LXXXII | 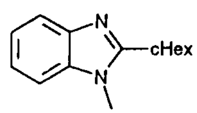 | (R&S) cPent | 99 | 0.44 (L) | 92 (Schaum) | 417 (100%) |

| Bsp.-Nr. | D | $R^1$ | Ausbeute (% d.Th.) | $R_f$ (Solvens) | Fp. (°C) | MS (*) (rel. Intensität) |
|---|---|---|---|---|---|---|
| LXXXIII | 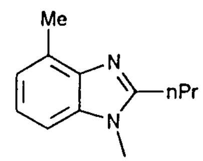 | (R&S) cPent | 89 | 0.10 (K) | | |
| LXXXIV | 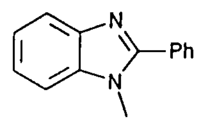 | (R&S) cPent | 81 | 0.30 (L) | | |
| LXXXV | 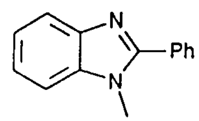 | (R&S) cHept | 97 | 0.57 (L) | 113 (Schaum) | * 438 (100%) |
| LXXXVI | 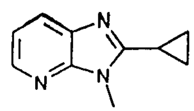 | (R&S) cPent | 71 | 0.25 (K) | | 376 (100%), 160 (40%) |
| LXXXVII | 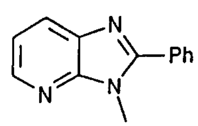 | (R&S) cPent | 46 | 0.07 (L) | | |

| Bsp.-Nr. | D | $R^1$ | Ausbeute (% d.Th.) | $R_f$ (Solvens) | Fp. (°C) | MS (*) (rel. Intensität) |
|---|---|---|---|---|---|---|
| LXXXVIII | 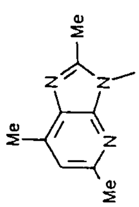 | (R&S) cPent | 96 | 0.24 (K) | >230 | 378 (100%), 154 (60%) |
| LXXXIX | 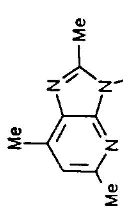 | (R&S) cHex | 71 | 0.27 (K) | 120 (Schaum) | 392 (100%) |
| XC | 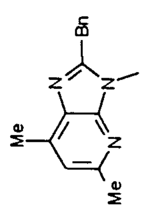 | (R&S) cPent | 87 | 0.16 (K) | 205 | #454 (100%) |
| XCI | 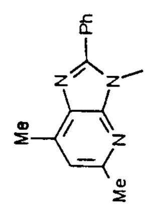 | (R&S) cPent | 37 | 0.37 (K) | 160 | 440 (100%) |

In Analogie zu den Vorschriften der Beispiele XXIII und LiX werden die in der Tabelle VI aufgeführten Beispiele hergestellt.

Tabelle VI:

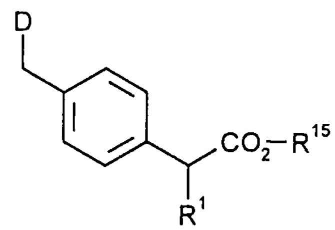

| Bsp.-Nr. | D | $R^1$ | $R^{15}$ | Fp. (°C) | Ausbeute (% d.Th.) | $R_f$ (Solvens) | MS (rel. Intensität) |
|---|---|---|---|---|---|---|---|
| XCII | 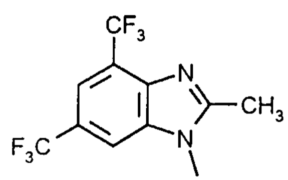 | (R&S) cPent | tBu | - | 30 | 0,37 (E) | 541 (100%)<br>57 ( 60%) |
| XCIII | 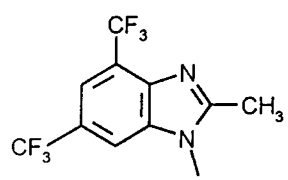 | (R&S) cPent | H | >220 | 84 | 0,09(K) | 485 (100%) |

**Herstellungsbeispiele**

**Beispiel 1**

2-(R)- und 2-(S)- 2-[4-(2-Phenyl-1H-benzimidazol-1-yl-methyl)-phenyl]-2-cyclopentyl-essigsäure-(2-(R)-2-phenyl-glycinol)-amid

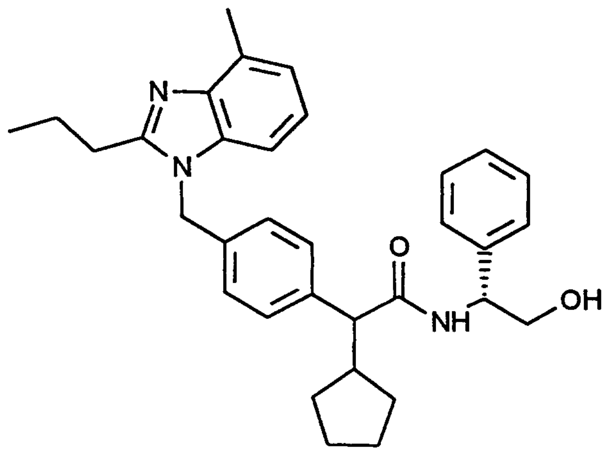

500 mg (1.22 mmol) der Verbindung aus Beispiel LXXXIII und 0.68 ml Triethylamin werden ei -30°C in 15 ml wasserfreiem N,N-Dimethylformamid 30 Minuten mit 0.10 ml Mesylchlorid umgesetzt. Darauf gibt man bei -30°C eine Lösung von 201 mg (1.46 mmol) 2-(R)-2-Phenyl-glycinol und 149 mg (1.22 mmol) 4-(N,N-dimethylamino)-pyridin in 15 ml wasserfreiem N,N-Dimethylformamid zu und rührt eine Stunde bei der genannten Temperatur nach. Nach einer Gesamtrührzeit von 20 Stunden unter allmählicher Erwärmung auf ca. 20°C gießt man auf Essigsäureethylester und wäßrige Natriumhydrogencarbonatlösung, trennt die Phasen und wäscht die wäßrige Lösung zweimal mit Essigsäureethylester nach. Die vereinigten organischen Phasen werden dann nacheinander mit Wasser (2 x), 1 M Natronlauge (3 x), Wasser (1 x) und Puffer von pH = 2 (2 x) extrahiert, mit wasserfreiem Natriumsulfat getrocknet und - zuletzt im Hochvakuum - eingedampft:

Ausbeute: 640 mg (1.2 mmol / 99%);
$R_f$ = 0.46 (Dichlormethan : Methanol = 10 : 1).

**Beispiel 2**

2-Cycloheptyl-N-(2-hydroxy-1-(R)-phenylethyl)-2-[4-(2,3-dimethylindol-1-yl-methyl)phenyl]essigsäureamid

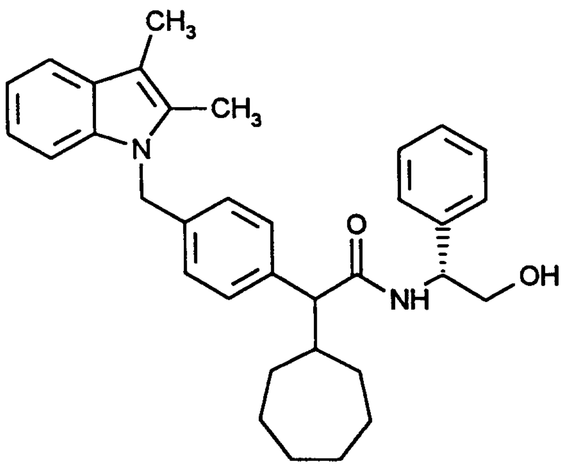

Es werden 538 mg (1.38 mmol) der Verbindung aus Beispiel LIX in 10 ml $CH_2Cl_2$ gelöst. Dazu gibt man 265 mg (1.38 mmol) R-Phenylglycinol. Die Lösung wird auf 0°C abgekühlt, anschließend versetzt man mit 204 mg (1.51 mmol) 1-Hydroxy-1-benzotriazol und 304 mg (1.58 mmol) N'-(3-Dimethylaminopropyl)-N-ethylcarbodiimid x HCl. Es wird dazu 279 mg (2.76 mmol) Triethylamin zugetropft. Man rührt über Nacht bei Raumtemperatur nach. Es wird mit Wasser und $CH_2Cl_2$ versetzt und extrahiert. Die organische Phase wird über Natriumsulfat getrocknet und einrotiert. Der Rückstand wird säulenchromatographisch gereinigt (Kieselgel Merck 60 (0.004 - 0.063)).

Ausbeute: 600 mg (85.4%);
Fp.: 163°C;
$R_f$ = 0.77 ($CH_2Cl_2$ : Methanol = 10 : 1);
Masse berechnet für $C_{34}N_{40}N_2O_2$ = 508.71; Massenspektrum (DCI ($NH_3$), rel. Intensität) 509 (50%), 508 (100%).

In Analogie zu den Vorschriften der Beispiele 1 und 2 werden die in der Tabelle 1 aufgeführten Verbindungen hergestellt:

Tabelle 1:

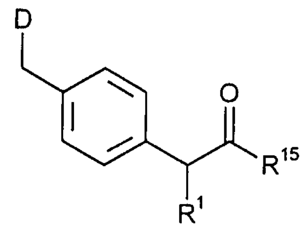

| Bsp.-Nr. | D | $R^1$ | $R^{15}$ | Ausbeute (% d.Th.) | $R_f$ (Solvens) | Fp. (°C) | MS (*) (rel. Intensität) |
|---|---|---|---|---|---|---|---|
| 3 | 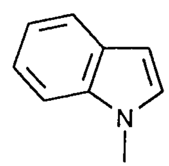 | (R&S) cHept | 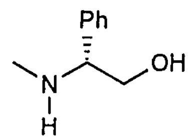 | 75 | 0.69 (L) | 170 | 481 (100%) |
| 4 | 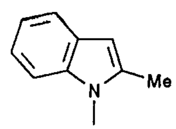 | (R&S) cHept | 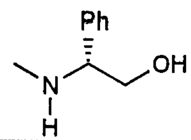 | 76 | 0.71 (L) | 163 | 495 (100%) |
| 5 | 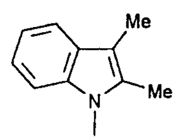 | (R&S) cPent | 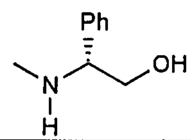 | 57 | | | 481 (80%)<br>480 (100%) |
| 6 | 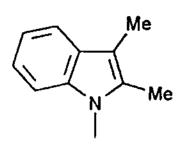 | (dia A) cPent | 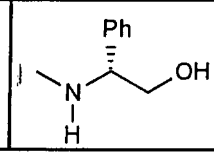 | | 0.37 (J) | 185 | |

| Bsp.-Nr. | D | $R^1$ | $R^{15}$ | Ausbeute (% d.Th.) | $R_f$ (Solvens) | Fp. (°C) | MS (*) (rel. Intensität) |
|---|---|---|---|---|---|---|---|
| 7 | 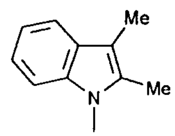 | (dia B) cPent | 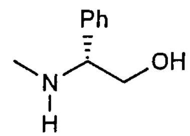 | | 0.23 (J) | 182 | |
| 8 | 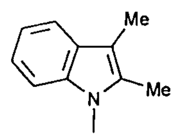 | (R&S) cHex | 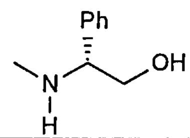 | 72 | 0.58 (J) | 180-82 | 494 (100%) |
| 9 | 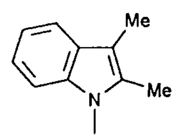 | (dia A) cHex | 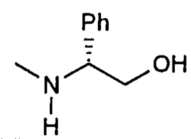 | | 0.54 (K) | 198 | |
| 10 | 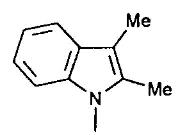 | (dia B) cHex | 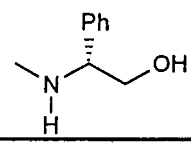 | | 0.39 (K) | 204 | |
| 11 | 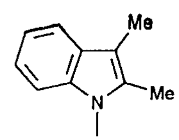 | (R&S) cHex | 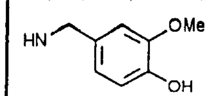 | 46 | 0.37 (J) | 98 (Schaum) | 510 (100%) |
| 12 | 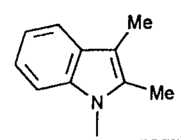 | (dia A) cHex | 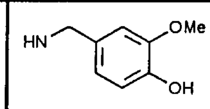 | | | 195-96 | |
| 13 | 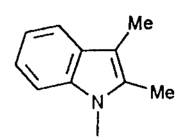 | (dia B) cHex | 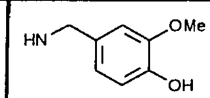 | | | 195-96 | |

| Bsp.-Nr. | D | $R^1$ | $R^{15}$ | Ausbeute (% d.Th.) | $R_f$ (Solvens) | Fp. (°C) | MS (*) (rel. Intensität) |
|---|---|---|---|---|---|---|---|
| 14 | 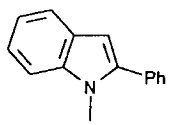 | (dia A) cPent | 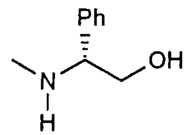 | 11 | 0.60 (K) | 183-85 | 529 (100%), 528 (80%) |
| 15 | 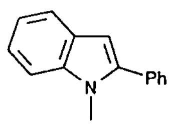 | (dia B) cPent | 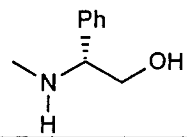 | 5 | 0.45 (K) | | 529 (100%), 528 (80%) |
| 16 | 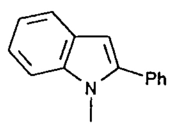 | (R&S) cHept | 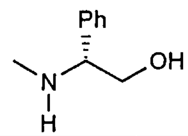 | 70 | 0.54 (K) | 182-86 | 557 (100%) |
| 17 | 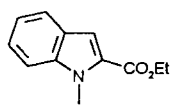 | (R&S) cPent | 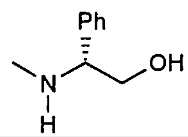 | 82 | | 118 | 525 (100%), 171 (100%) |
| 18 | 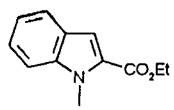 | (dia A) cPent | 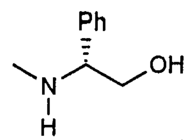 | | 0.54 (K) | | |
| 19 | 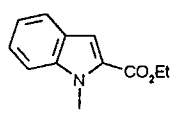 | (dia B) cPent | 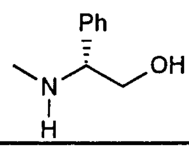 | | 0.44 (K) | | |
| 20 | 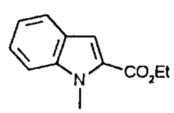 | (R&S) cHept | 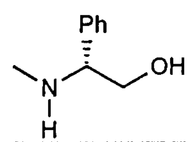 | 81 | 0.52 (K) | 178-81 | 553 (100%), 199 (100%) |

| Bsp.-Nr. | D | $R^1$ | $R^{15}$ | Ausbeute (% d.Th.) | $R_f$ (Solvens) | Fp. (°C) | MS (*) (rel. Intensität) |
|---|---|---|---|---|---|---|---|
| 21 | 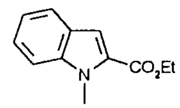 | (dia A) cHept | 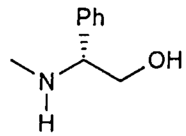 | | | 204 | |
| 22 | 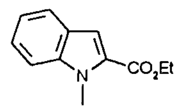 | (dia B) cHept | 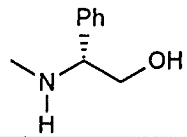 | | | 203 | |
| 23 | 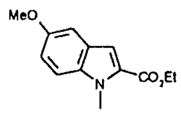 | (R&S) cPent | 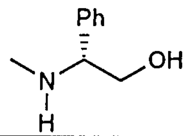 | 61 | | 116 | 555 (100%), 554 (90%) |
| 24 | 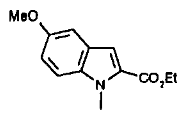 | (dia A) cPent | 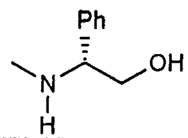 | | 0.47 (K) | | |
| 25 | 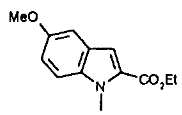 | (dia B) cPent | 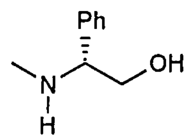 | | 0.38 (K) | | |
| 26 | 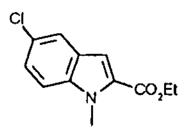 | (R&S) cPent | 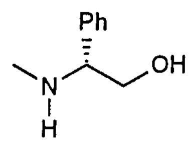 | 83 | | 158 | 559 (60%), 171 (100%) |
| 27 | 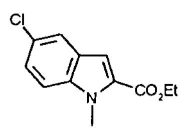 | (dia A) cPent | 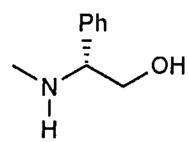 | | 0.48 (K) | 162-65 | |

| Bsp.-Nr. | D | $R^1$ | $R^{15}$ | Ausbeute (% d.Th.) | $R_f$ (Solvens) | Fp. (°C) | MS (*) (rel. Intensität) |
|---|---|---|---|---|---|---|---|
| 28 | 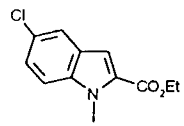 | (dia B) cPent | 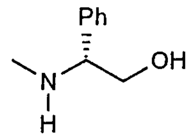 | | 0.38 (K) | 191-93 | |
| 29 | 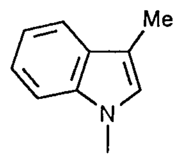 | (R&S) cHept | 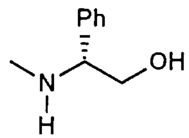 | 85 | 0.73 (L) | 171 | # 494 (100%) |
| 30 | 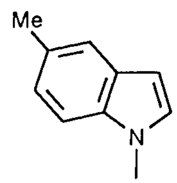 | (R&S) cPent | 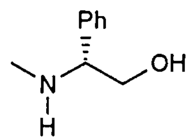 | 50 | 0.53 (K) | 155-61 | 467 (100%), 466 (80%) |
| 31 | 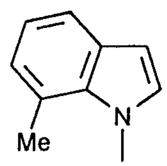 | (R&S) cPent | 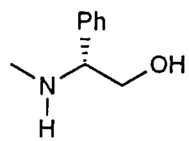 | 78 | 0.46 (K) | 186-90 | 467 (100%), 466 (80%) |
| 32 | 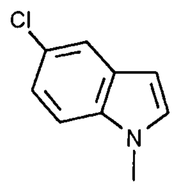 | (R&S) cHept | 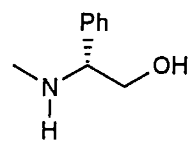 | 66 | 0.47 (K) | 153-55 | 515 (100%), 164 (80%) |
| 33 | 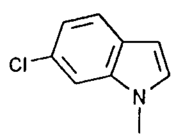 | (R&S) cPent | 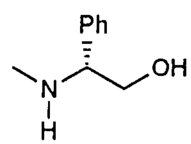 | 38 | 0.59 (K) | 170-75 | 487 (100%) |

| Bsp.-Nr. | D | $R^1$ | $R^{15}$ | Ausbeute (% d.Th.) | $R_f$ (Solvens) | Fp. (°C) | MS (*) (rel. Intensität) |
|---|---|---|---|---|---|---|---|
| 34 | 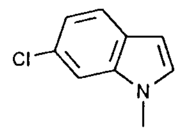 | (dia A) cPent | 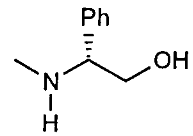 | | | 160-62 | |
| 35 | 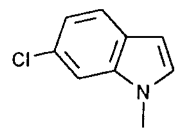 | (dia B) cPent | 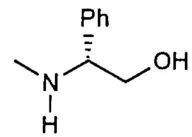 | | | 204-05 | |
| 36 | 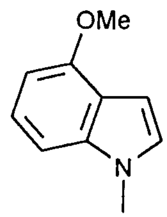 | (R&S) cPent | 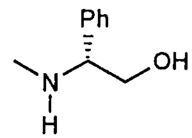 | 71 | 0.52 (K) | 173-76 | 482 (100%) |
| 37 | 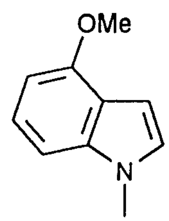 | (dia A) cPent | 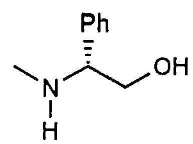 | | | 183-85 | |
| 38 | 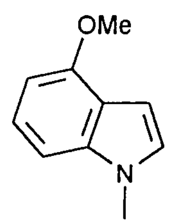 | (dia B) cPent | 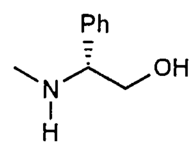 | | | 200-03 | |

| Bsp.-Nr. | D | $R^1$ | $R^{15}$ | Ausbeute (% d.Th.) | $R_f$ (Solvens) | Fp. (°C) | MS (*) (rel. Intensität) |
|---|---|---|---|---|---|---|---|
| 39 | 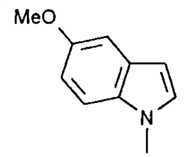 | (R&S) cHept | 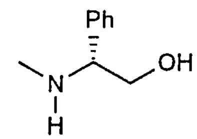 | 78 | 0.55 (K) | 163-68 | 511 (80%), 510 (100%) |
| 40 | 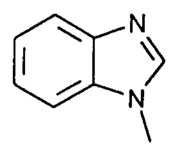 | (R&S) cHept | 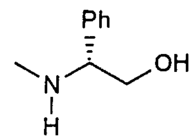 | 86 | 0.25 (K) | 108 (Schaum) | # 482 (100%) |
| 41 | 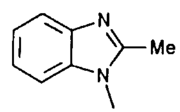 | (R&S) cHept | 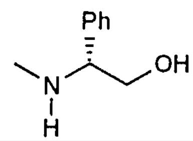 | 69 | 0.03 (K) | 110 (Schaum) | # 496 (100%) |
| 42 | 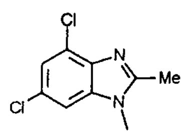 | (R&S) cPent | 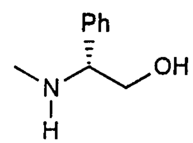 | 54 | 0.28 (K) | 230-33 | 538 (70%), 536 (100%) |
| 43 | 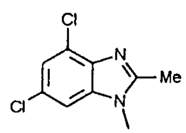 | (dia A) cPent | 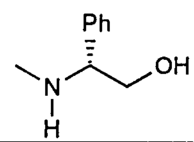 | | | | |
| 44 | 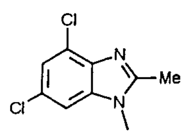 | (dia B) cPent | 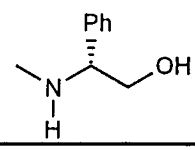 | | | >220 | |

| Bsp.-Nr. | D | $R^1$ | $R^{15}$ | Ausbeute (% d.Th.) | $R_f$ (Solvens) | Fp. (°C) | MS (*) (rel. Intensität) |
|---|---|---|---|---|---|---|---|
| 45 | 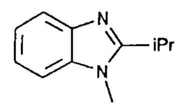 | (R&S) cPent | 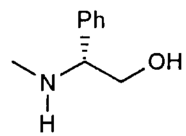 | 83 | 0.32 (K) | 93 | 496 (100%) |
| 46 | 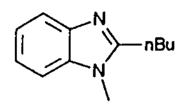 | (R&S) cPent | 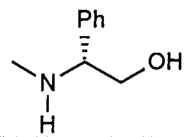 | 88 | 0.52 (L) | | |
| 47 | 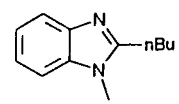 | (dia A) cPent | 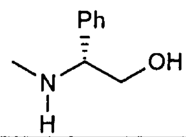 | | 0.52 (L) | | |
| 48 | 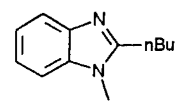 | (dia B) cPent | 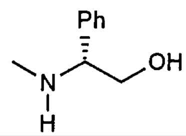 | | 0.52 (L) | | |
| 49 | 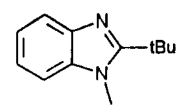 | (R&S) cPent | 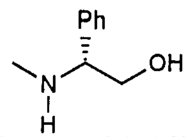 | 76 | 0.37 (K) | 104 (Schaum) | 510 (100%), 154 (60%) |
| 50 | 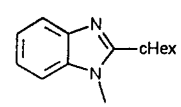 | (R&S) cPent | 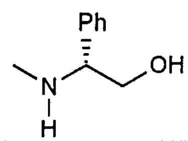 | 90 | 0.52 (K) | 100 (Schaum) | 536 (100%), 105 (55%) |
| 51 | 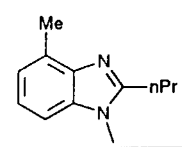 | (R&S) cPent | 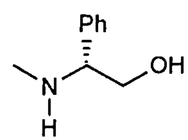 | 54 | 0.47 (L) | | 510 (100%) |

| Bsp.-Nr. | D | $R^1$ | $R^{15}$ | Ausbeute (% d.Th.) | $R_f$ (Solvens) | Fp. (°C) | MS (*) (rel. Intensität) |
|---|---|---|---|---|---|---|---|
| 52 | 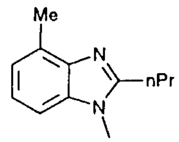 | (dia A) cPent | 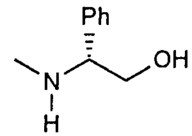 | | 0.47 (L) | | |
| 53 | 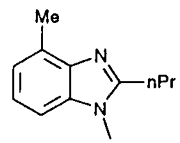 | (dia B) cPent | 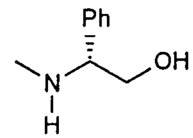 | | 0.43 (L) | | |
| 54 | 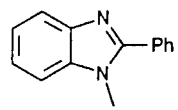 | (R&S) cPent | 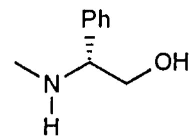 | 99 | 0.54 (L) | | 530 (100%) |
| 55 | 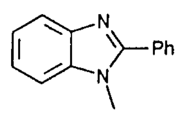 | (dia A) cPent | 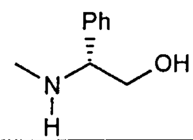 | | 0.54 (L) | | |
| 56 | 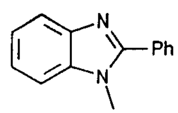 | (dia B) cPent | 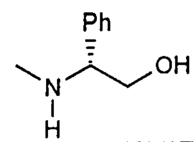 | | 0.50 (L) | | |
| 57 | 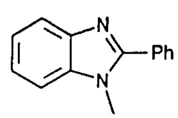 | (R&S) cHept | 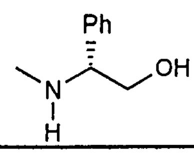 | 41 | 0.15 (U) | amorph | 558 (100%) |
| 58 | 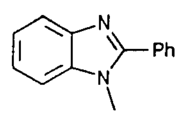 | (dia A) cHept | 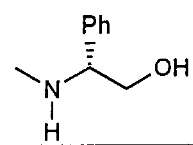 | | | 191-2 | |

| Bsp.-Nr. | D | $R^1$ | $R^{15}$ | Ausbeute (% d.Th.) | $R_f$ (Solvens) | Fp. (°C) | MS (*) (rel. Intensität) |
|---|---|---|---|---|---|---|---|
| 59 | 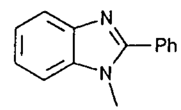 | (dia B) cHept | 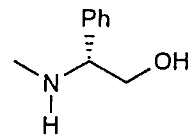 | | | 211-2 | |
| 60 | 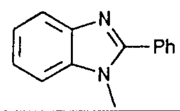 | (R&S) cHept | 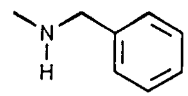 | 56 | 0.31 (U) | amorph | 426 (100%) |
| 61 | 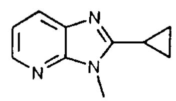 | (R&S) cPent | 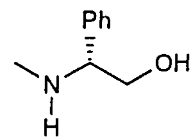 | 65 | 0.26 (K) | | 495 (100%) |
| 62 | 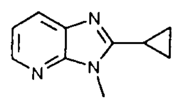 | (dia A) cPent | 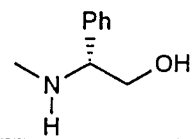 | | | 172 | |
| 63 | 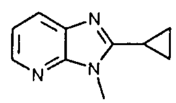 | (dia B) cPent | 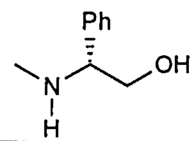 | | | 213 | |
| 64 | 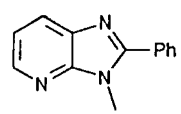 | (R&S) cPent | 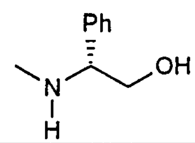 | 86 | 0.23 (K) | | 531 (100%) |
| 65 | 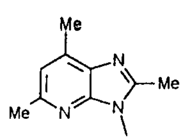 | (R&S) cPent | 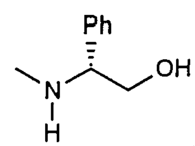 | 75 | | | 497 (100%) |

| Bsp.-Nr. | D | $R^1$ | $R^{15}$ | Ausbeute (% d.Th.) | $R_f$ (Solvens) | Fp. (°C) | MS (*) (rel. Intensität) |
|---|---|---|---|---|---|---|---|
| 66 | 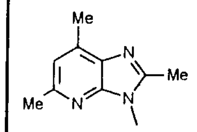 | (dia A) cPent | 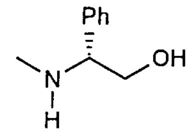 | | 0.20 (K) | 106-108 (Schaum) | |
| 67 | 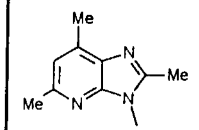 | (dia B) cPent | 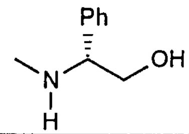 | | 0.16 (K) | 234 | |
| 68 | 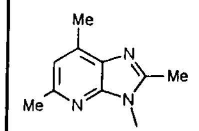 | (R&S) cHex | 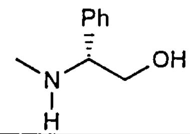 | | | | 511 (100%) |
| 69 | 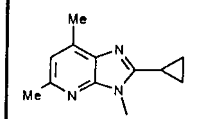 | (R&S) cPent | 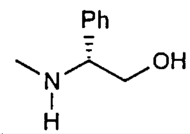 | 47 | 0.47 (K) | 186 | 523 (60%), 55 (100%) |
| 70 | 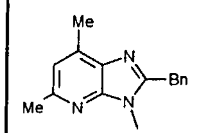 | (R&S) cPent | 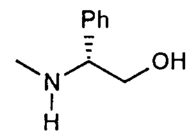 | 76 | 0.60 (K) | 100 (Schaum) | #573 (100%) |
| 71 | 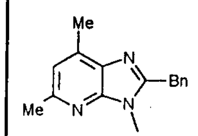 | (dia A) cPent | 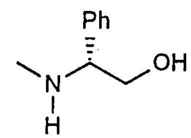 | | 0.41 (K) | 100 | |
| 72 | 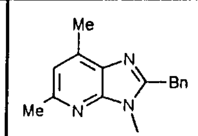 | (dia B) cPent | 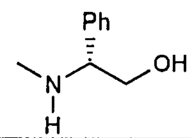 | | 0.35 (K) | 150 | |

| Bsp.-Nr. | D | $R^1$ | $R^{15}$ | Ausbeute (% d.Th.) | $R_f$ (Solvens) | Fp. (°C) | MS (*) (rel. Intensität) |
|---|---|---|---|---|---|---|---|
| 73 | 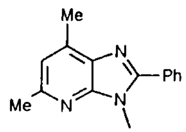 | (R&S) cPent | 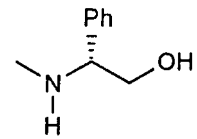 | 72 | 0.44 (K) | 210 | 559 (100%) |
| 74 | 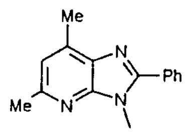 | (dia A) cPent | 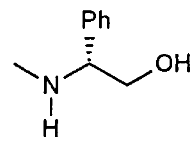 | | | | |
| 75 | 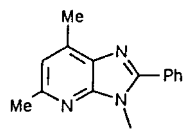 | (dia B) cPent | 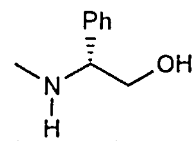 | | | | |
| 76 | 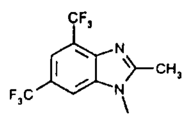 | (R&S) cPent | 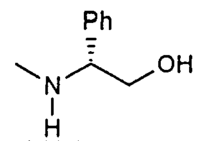 | 82 | 210-12 | 604 (60%), 105 (100%) | |
| 77 | 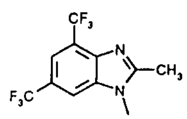 | (dia A) cPent | 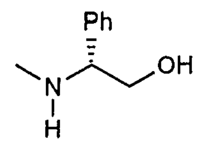 | | 229 | | |
| 78 | 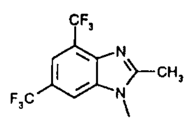 | (dia B) cPent | 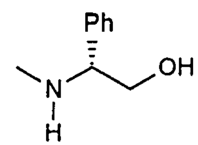 | | 212 | | |

**Patentansprüche**

1. Indolyl-substituierte Phenylessigsäurederivate der allgemeinen Formel (I)

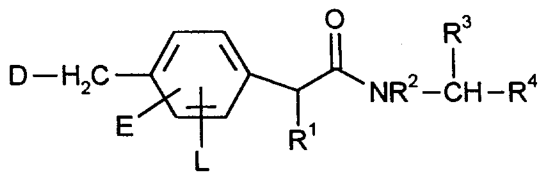 (I)

in welcher

D für einen Rest der Formel

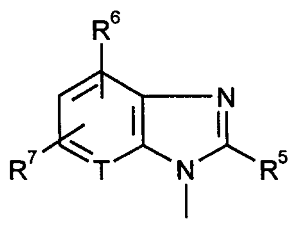 oder 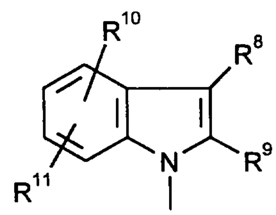

steht,
worin

T ein Stickstoffatom oder die -CH-Gruppe bedeutet,

$R^6$, $R^7$, $R^{10}$ und $R^{11}$ gleich oder verschieden sind und Wasserstoff, Trifluormethyl, Halogen oder geradkettiges oder verzweigtes Alkyl oder Alkoxy mit jeweils bis zu 6 Kohlenstoffatomen bedeuten,

$R^5$, $R^8$ und $R^9$ gleich oder verschieden sind und Wasserstoff, Cycloalkyl mit 3 bis 6 Kohlenstoffatomen, Phenyl, geradkettiges oder verzweigtes Alkoxycarbonyl mit bis zu 6 Kohlenstoffatomen oder geradkettiges oder verzweigtes Alkyl mit bis zu 6 Kohlenstoffatomen bedeuten, das gegebenenfalls durch Halogen substituiert ist,

oder im Fall, daß T für ein Stickstoffatom steht, $R^5$ auch Benzyl bedeuten kann,

E und L gleich oder verschieden sind und für Wasserstoff, Halogen, Trifluormethyl, Hydroxy, Carboxyl oder für geradkettiges oder verzweigtes Alkyl, Alkoxy oder Alkoxycarbonyl mit jeweils bis zu 6 Kohlenstoffatomen stehen,

$R^1$ für Cycloalkyl mit 3 bis 10 Kohlenstoffatomen oder für geradkettiges oder verzweigtes Alkyl mit 1 bis 10 Kohlenstoffatomen steht, oder für Phenyl steht, das gegebenenfalls bis zu 2-fach gleich oder verschieden durch Halogen, Cyano, Hydroxy, geradkettiges oder verzweigtes Alkyl oder Alkoxy mit jeweils bis zu 4 Kohlenstoffatomen substituiert ist,

$R^2$ für Wasserstoff oder geradkettiges oder verzweigtes Alkyl mit bis zu 3 Kohlenstoffatomen steht,

$R^3$ für Wasserstoff oder geradkettiges oder verzweigtes Alkyl mit bis zu 5 Kohlenstoffatomen steht, oder für Cycloalkyl mit 3 bis 7 Kohlenstoffatomen steht, oder

für Phenyl oder für einen 5- bis 7-gliedrigen aromatischen Heterocyclus mit bis zu 3 Heteroatomen aus der Reihe S, N und/oder O steht, die gegebenenfalls bis zu 3-fach gleich oder verschieden durch Halogen, Nitro, Phenyl, Hydroxy oder durch geradkettiges oder verzweigtes Alkyl oder Alkoxy mit bis zu 6 Kohlenstoffatomen substituiert sind,

$R^4$ für Wasserstoff oder für eine Gruppe der Formel $-CH_2-OH$ oder $CH_2O-CO-R^{12}$ steht,

worin

$R^{12}$ Wasserstoff, geradkettiges oder verzweigtes Alkyl mit bis zu 8 Kohlenstoffatomen oder Phenyl bedeutet, das gegebenenfalls bis zu 3-fach gleich oder verschieden durch Halogen, Hydroxy, Cyano oder geradkettiges oder verzweigtes Alkyl oder Alkoxy mit jeweils bis zu 4 Kohlenstoffatomen substituiert ist,

und deren Salze.

2. Indolyl-substituierte Phenylessigsäurederivate der Formel nach Anspruch 1, in welcher

D für einen Rest der Formel

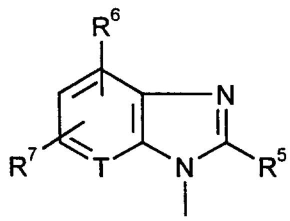

oder

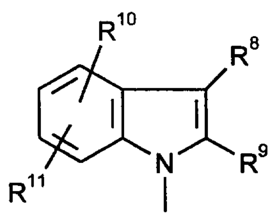

steht,
worin

T ein Stickstoffatom oder die -CH-Gruppe bedeutet,

$R^6$, $R^7$, $R^{10}$ und $R^{11}$ gleich oder verschieden sind und Wasserstoff, Trifluormethyl, Fluor, Chlor, Brom oder geradkettiges oder verzweigtes Alkyl oder Alkoxy mit jeweils bis zu 5 Kohlenstoffatomen bedeuten,

$R^5$, $R^8$ und $R^9$ gleich oder verschieden sind und Wasserstoff, Cyclopropyl, Cyclopentyl, Cyclohexyl, Phenyl, geradkettiges oder verzweigtes Alkoxycarbonyl mit bis zu 5 Kohlenstoffatomen oder geradkettiges oder verzweigtes Alkyl mit bis zu 5 Kohlenstoffatomen bedeuten, das gegebenenfalls durch Fluor, Chlor oder Brom substituiert ist,

oder im Fall, daß T für ein Stickstoffatom steht, $R^5$ auch Benzyl bedeuten kann,

E und L gleich oder verschieden sind und für Wasserstoff, Fluor, Chlor, Brom, Trifluormethyl, Hydroxy oder geradkettiges oder verzweigtes Alkyl oder Alkoxy mit jeweils bis zu 4 Kohlenstoffatomen stehen,

$R^1$ für Cyclobutyl, Cyclopentyl, Cyclohexyl, Cycloheptyl, Cylcooctyl oder für geradkettiges oder verzweigtes Alkyl mit bis zu 7 Kohlenstoffatomen steht, oder für Phenyl steht, das gegebenenfalls durch Fluor, Chlor, Brom, Cyano, Hydroxy oder geradkettiges oder verzweigtes Alkyl oder Alkoxy mit jeweils bis zu 3 Kohlenstoffatomen substituiert ist,

$R^2$ Wasserstoff oder Methyl bedeutet,

$R^3$ für Wasserstoff oder geradkettiges oder verzweigtes Alkyl mit bis zu 4 Kohlenstoffatomen, Cyclopropyl, Cyclopentyl, Cyclohexyl oder für Phenyl, Pyridyl, Thienyl oder Furyl steht, die gegebenenfalls bis zu 2-fach gleich oder verschieden durch Fluor, Chlor, Brom, Phenyl, Nitro, Hydroxy oder durch geradkettiges oder verzweigtes Alkyl oder Alkoxy mit bis zu 4 Kohlenstoffatomen substituiert sind,

$R^4$ für Wasserstoff oder für eine Gruppe der Formel $-CH_2-OH$ oder $-CH_2O-CO-R^{12}$ steht,

worin

$R^{12}$ Wasserstoff, geradkettiges oder verzweigtes Alkyl mit bis zu 6 Kohlenstoffatomen oder Phenyl bedeutet, das gegebenenfalls bis zu 2-fach gleich oder verschieden durch Fluor, Chlor, Brom, Cyano, Hydroxy oder geradkettiges oder verzweigtes Alkyl oder Alkoxy mit jeweils bis zu 3 Kohlenstoffatomen substituiert ist,

und deren Salze.

**3.** Indolyl-substituierte Phenylessigsäurederivate der Formel nach Anspruch 1, in welcher

D für einen Rest der Formel

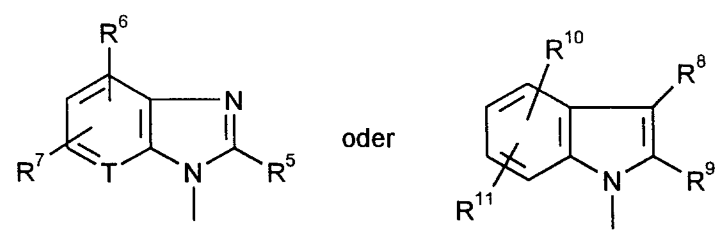

steht,

worin

T ein Stickstoffatom oder die -CH-Gruppe bedeutet,

$R^6$, $R^7$, $R^{10}$ und $R^{11}$ gleich oder verschieden sind und Wasserstoff, Trifluormethyl, Chlor oder geradkettiges oder verzweigtes Alkyl oder Alkoxy mit jeweils bis zu 4 Kohlenstoffatomen bedeuten,

$R^5$, $R^8$ und $R^9$ gleich oder verschieden sind und Wasserstoff, Cyclopropyl, Cyclopentyl, Cyclohexyl, Phenyl, geradkettiges oder verzweigtes Alkoxycarbonyl mit bis zu 4 Kohlenstoffatomen oder geradkettiges oder verzweigtes Alkyl mit bis zu 4 Kohlenstoffatomen bedeuten, das gegebenenfalls durch Chlor substituiert ist,

oder im Fall, daß T für ein Stickstoffatom steht, $R^5$ auch Benzyl bedeuten kann,

E und L gleich oder verschieden sind und für Wasserstoff, Fluor, Chlor, Brom oder Trifluormethyl stehen,

$R^1$ für Cyclopentyl, Cyclohexyl, Cycloheptyl, Cylcooctyl oder für geradkettiges oder verzweigtes Alkyl mit bis zu 6 Kohlenstoffatomen steht, oder für Phenyl steht, das gegebenenfalls durch Fluor, Chlor, Cyano, Hydroxy, Methyl oder Methoxy substituiert ist,

$R^2$ für Wasserstoff oder Methyl steht,

$R^3$ für Wasserstoff oder geradkettiges oder verzweigtes Alkyl mit bis zu 3 Kohlenstoffatomen, Cyclopropyl,

Cyclopentyl, Cyclohexyl oder für Pyridyl, Thienyl oder Phenyl steht, die gegebenenfalls bis zu 2-fach gleich oder verschieden durch Fluor, Chlor, Brom, Nitro, Phenyl, Hydroxy oder durch geradkettiges oder verzweigtes Alkyl oder Alkoxy mit bis zu 3 Kohlenstoffatomen substituiert sind,

$R^4$ für Wasserstoff, für eine Gruppe der Formel $-CH_2-OH$ oder $-CH_2O-CO-R^{12}$ steht,

worin

$R^{12}$ Wasserstoff, geradkettiges oder verzweigtes Alkyl mit bis zu 6 Kohlenstoffatomen oder Phenyl bedeutet, das gegebenenfalls bis zu 2-fach gleich oder verschieden durch Fluor, Chlor, Brom, Cyano, Hydroxy, Methyl oder Methoxy substituiert ist,

und deren Salze.

**4.** Indolyl-substituierte Phenylessigsäurederivate nach Anspruch 1 bis 3 zur therapeutischen Anwendung.

**5.** Verfahren zur Herstellung von Indolyl-substituierten Phenylessigsäurederivaten nach Anspruch 1 bis 3, dadurch gekennzeichnet, daß man
Verbindungen der allgemeinen Formel (II)

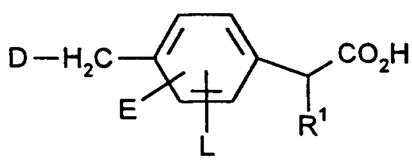

(II)

in welcher

D, E, L und $R^1$ die oben angegebene Bedeutung haben,

mit Verbindungen der allgemeinen Formel (III)

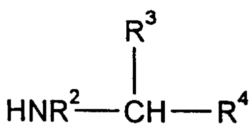

(III)

in welcher

$R^2$, $R^3$ und $R^4$ die oben angegebene Bedeutung haben,

in inerten Lösemitteln, gegebenenfalls in Anwesenheit einer Base und/oder Hilfsstoffes umsetzt.

**6.** Arzneimittel enthaltend Indolyl-substituierte Phenylessigsäurederivate nach Anspruch 1 bis 3.

**7.** Arzneimittel nach Anspruch 6 zur Behandlung von Arteriosklerose.

**8.** Verfahren zur Herstellung von Arzneimitteln nach Anspruch 6 und 7, dadurch gekennzeichnet, daß man die Indolyl-substituierten Phenylessigsäurederivate gegebenenfalls mit üblichen Hilfs- und Trägerstoffen in eine geeignete Applikationsform überführt.

**9.** Verwendung der Indolyl-substituierten Phenylessigsäurederivate nach Anspruch 1 bis 3 zur Herstellung von Arzneimitteln.

**10.** Verwendung nach Anspruch 9 zur Herstellung von antiarteriosklerotisch wirksamen Arzneimitteln.

Europäisches Patentamt

# EUROPÄISCHER RECHERCHENBERICHT

Nummer der Anmeldung
EP 96 11 9320

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A<br><br>D | EP 0 610 698 A (BAYER AG)<br>* das ganze Dokument *<br>& DE 43 02 956 A<br>--- | 1,6 | C07D209/42<br>C07D235/16<br>C07D235/18<br>C07D471/04<br>A61K31/415 |
| A | EP 0 560 162 A (BAYER AG)<br>* das ganze Dokument *<br>--- | 1,6 | |
| A | EP 0 560 163 A (BAYER AG)<br>* das ganze Dokument *<br>--- | 1,6 | |
| A | EP 0 565 986 A (BAYER AG)<br>* das ganze Dokument *<br>--- | 1,6 | |
| A<br><br>D | EP 0 513 533 A (BAYER AG)<br>* das ganze Dokument *<br>& DE 42 00 954 A<br>* das ganze Dokument *<br>----- | 1,6 | |
| | | | RECHERCHIERTE SACHGEBIETE (Int.Cl.6) |
| | | | C07D |

Der vorliegende Recherchenbericht wurde für alle Patentansprüche erstellt

| Recherchenort | Abschlußdatum der Recherche | Prüfer |
|---|---|---|
| BERLIN | 21.März 1997 | Kyriakakou, G |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)